(19) Europäisches Patentamt
European Patent Office
Office européen des brevets

(11) Publication number: **0 335 902 B1**

# (12) EUROPEAN PATENT SPECIFICATION

(45) Date of publication of patent specification: **01.12.93** (51) Int. Cl.5: **G01N 33/533**

(21) Application number: **88900427.1**

(22) Date of filing: **11.12.87**

(86) International application number:
**PCT/US87/03226**

(87) International publication number:
**WO 88/04777 (30.06.88 88/14)**

The file contains technical information submitted after the application was filed and not included in this specification

## (54) MONOMERIC PHTHALOCYANINE REAGENTS.

(30) Priority: **15.12.86 US 941619**
**24.12.86 US 946475**
**12.06.87 US 61937**

(43) Date of publication of application:
**11.10.89 Bulletin 89/41**

(45) Publication of the grant of the patent:
**01.12.93 Bulletin 93/48**

(84) Designated Contracting States:
**AT BE CH DE FR GB IT LI LU NL SE**

(56) References cited:
**EP-A- 0 063 852**
**EP-A- 0 142 369**
**US-A- 4 160 645**
**US-A- 4 277 437**
**US-A- 4 560 534**

(73) Proprietor: **BRITISH TECHNOLOGY GROUP USA INC.**
**Renaissance Business Park**
**2200 Renaissance Boulevard**
**Gulph Mills Pennsylvania 19406(US)**

(72) Inventor: **STANTON, Thomas, H.**
**528 NE 82nd**
**Seattle, WA 98115(US)**
Inventor: **SCHINDELE, Deborah, C.**
**15019 59th Place NE**
**Bothell, WA 98011(US)**
Inventor: **RENZONI, George, E.**
**15019 59th Place NE**
**Bothell, WA 98011(US)**
Inventor: **PEPICH, Barry, V.**
**4735 22nd NE, 2**
**Seattle, WA 98105(US)**

EP 0 335 902 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 99, 1983, Columbus, OH (US); T. HARA et al., p. 320, no. 172173u&NUM;**

**CHEMICAL ABSTRACTS, vol. 103, 1985, Columbus, OH (US); T. HARA etal., p. 301, no. 84292c&NUM;**

**BULLETIN OF THE CHEMICAL SOCIETY OF JAPAN, vol. 56, 1983; T. HARA et al., pp. 2965-2968&NUM;**

**INORGANIC CHEMISTRY, vol. 30, 1991; P.C. MARTIN et al., pp. 3305-3309&NUM;**

Inventor: **ANDERSON, Neils, H.**
**8022 Burke Avenue North**
**Seattle, WA 98103(US)**
Inventor: **CLAGETT, James, A.**
**12803 25th Avenue SE**
**Everett, WA 98204(US)**
Inventor: **OPHEIM, Kent, E.**
**6049 51st NE**
**Seattle, WA 98115(US)**

(74) Representative: **Sheard, Andrew Gregory et al**
**Kilburn & Strode**
**30, John Street**
**London WC1N 2DD (GB)**

## Description

### Field of the Invention

The invention provides water-soluble metallated phthalocyanine compounds that are monomerically conjugated to biochemical moieties such as antibodies or enzyme-cleavable ligands for use either as detectable reagents in immunoassays and flow cytometry procedures or as therapeutic reagents for directed cell killing via singlet oxygen generation.

### Background of the Invention

The phthalocyanine pigments are a group of light-fast organic pigments with four isoindole groups, $(C_6H_4)C_2N$, linked by four nitrogen atoms to form a cyclic conjugated chain. Included are phthalocyanine (blue-green), copper phthalocyanine (blue), chlorinated copper phthalocyanine (green), and sulfonated copper phthalocyanine (green). These pigments are commonly used in enamels, plastics, linoleum, inks, wallpaper, fabrics, paper, and rubber goods.

Free base phthalocyanine, and aluminum, cadmium, magnesium, silicon, tin, and zinc metallated phalocyanines are reported to be fluorescent; see *The Phthalocyanines 1*:127, 1983. One or more of these species have been utilized in or proposed for semiconductors, organic dyes, stain removing agents, bactericides, and optical coatings.

European patent publication No. 142,369 discloses the use of certain phthalocyanine derivatives for hematology, specifically to differentiate basophils from other blood cells.

Phthalocyanines have been reported for potential use in various immunoassays. See: U.S. Patent No. 4,160,645 (at column 18, lines 18 to 22); U.S. Patent No. 4,193,983 (at column 16, lines 36 to 39); U.S. Patent No. 4,220,450 (at column 17, lines 23 to 26); U.S. Patent No. 4,233,402 (at column 24, lines 53 to 56); U.S. Patent No. 4,235,869 (at column 11, line 67 to column 12, line 2); U.S. Patent No. 4,256,834 (at column 21, lines 34 to 36); U.S. Patent No. 4,277,437 (at column 17, lines 11 to 14); U.S. Patent No. 4,318,707 (at column 9, lines 14 to 16); U.S. Patent No. 4,483,929 (at column 6, lines 36 to 39); U.S. Patent No. 4,540,660; U.S. Patent No. 4,540,670 (at column 11, lines 40 to 59); U.S. Patent No. 4,560,534 (at column 5, line 67 to column 6, line 7); U.S. Patent No. 4,650,770 (at column 18, lines 22 to 25); U.S. Patent No. 4,656,129; and U.S. Patent No. 4,659,676 (at column 2, lines 40 to 46).

Phthalocyanine derivatives have been employed as catalysts in chemiluminescence immunoassay systems. See: Hara, T., et al., *Bull. Chem. Soc. Jpn. 56*:2965-2968, 1983; Hara, T., et al., *Bull. Chem. Soc. Jpn. 56*:2267-2271, 1983; Hare, T., et al., *Bull. Chem. Soc. Jpn. 57*:587-588, 1984; Hara, T., et al., *Bull. Chem. Soc. Jpn. 57*:3009-3010, 1984; and Hara, T., et al., *Bull. Chem. Soc. Jpn. 57*:3009-3010, 1984; and Hara, T., et al., *Bull. Chem. Soc. Jpn. 58*:1299-1303, 1985. Hara described a chemiluminescence complex catalyst immunoassay in which iron phthalocyanine serves as the catalyst for a chemiluminescent reaction between luminol and hydrogen peroxide. The chemiluminescent signal is quantitated and correlated with the amount of analyte present in the test sample. Hara examined a number of phthalocyanine (Fe, Co) and porphyrin (Fe, Pd, Pt, Mn, Sn) complexes and reported that iron phthalocyanine exhibits the greatest catalytic activity and provides the highest sensitivities for this type of assay. The resultant product of labelling proteins with phthalocyanine is highly aggregated.

The phthalocyanines have also been suggested for use in photodynamic therapy (PDT), which is a radiation therapy for cancer that utilizes a photosensitive agent (sensitizer) and visible light as the radiation source. The sensitizer must be selectively delivered to the tumor tissues; for example, monoclonal antibody-hematoporphyrin conjugates have been reported. Mew, D., et al., *J. Immunol. 130* (3):1473-1477, 1983. Thereafter, activation of the sensitizer by visible light kills the cells by a photodynamic reaction involving singlet oxygen generation. The phthalocyanines, particularly the aluminum and zinc tetrasulfonate derivatives, have been suggested for use in PDT, based upon their use as photosensitizers for cultured mammalian cells. See: Ben-Hur, E., and I. Rosenthal, *Int. J. Radiat. Biol. 47*:145-147, 1985; Ben-Hur, E., and I. Rosenthal, *Photochem. and Photobiol. 42*:129-133, 1985; Ben-Hur, E., and I. Rosenthal, *Radiat. Res. 103*:403-409, 1985, Brasseur, N., et al., *Photochem. and Photobiol. 42*:515-521, 1985; Ben-Hur, E., and I. Rosenthal, *Lasers in the Life Sciences 1*:79-86, 1986; Ben-Hur, E., I. Rosenthal, *Photochem. and Photobiol. 43*:615-621, 1986; Chan, W. S. et al., *Br. J. Cancer 53*:255-263, 1986; Rosenthal, I. et al., *Radiat. Res.* 107:136-142, 1986; Salman, S. H., et al., *J. Urology 136*:141-145, 1986; and Ben-Hur, E., et al., *Int. J. Radiat Biol. 51*:467-476, 1987.

Fluorescent compounds (fluorophores) have been widely used in immunoassays, flow cytometry, and fluorescence microscopy. In addition, the most sensitive enzymatic immunoassays employ fluorogenic

rather than colorimetric substrates. Three well-known fluorogenic enzyme substrate couples are: alkaline phosphatase (AP) and 4-methylumbelliferylphosphate (MUP); β-galactosidase (β-Gal) and 4-methylumbelliferyl-D-galactopyranoside (MUG); and horseradish peroxidase (HRP) and *p*-hydroxyphenyl acetic acid (HPA). Generally, the AP, β-Gal, and HRP systems are useful for detection of analytes at concentrations greater than $10^{-15}$ M. To date, the sensitivity of these systems is limited by the spectral properties of the generated fluorophores.

## Summary of the Invention

The invention provides fluorescent and/or chromogenic reagents in which a phthalocyanine derivative is monomerically conjugated with a antigen, antibody, or oligonucleotide. The monomeric as opposed to dimeric or more highly aggregated nature of the phthalocyanine moiety is critical to preserving the emissivity and/or absorptivity of the phthalocyanine in the reagent, and hence its signal strength and the sensitivity of an assay in which the subject reagents are employed.

For use as a fluorophore, the free base phthalocyanine may be metallated with aluminum, silicon, phosphorous, gallium, germanium, cadmium, magnesium, tin, or zinc. For use as a chromogen, the phthalocyanine may or may not be metallated. For use in aqueous solution, the phthalocyanine macrocycle should be derivatized with water-solubilizing substituents such as sulfonic acid, sulfonate, carboxylic acid, carboxylate, phosphoric acid, phosphate, phosphonate, hydroxy, phenoxy, amino, ammonium, and pyridinium substituents. To further promote disaggregation, metallation with an atom of +3 valence or higher is recommended, so that the phthalocyanine will take on an axial ligand in aqueous solution. Another way of preventing aggregation (and also nonspecific binding) is to conjugate the reagent with a hydrophilic component, like bovine serum albumin, that substantially envelops the phthalocyanine monomer.

The subject reagents are useful as detectable markers in immunoassays. For use in enzymatic immunoassays and cDNA assays, the monomeric phthalocyanine derivative is typically conjugated via an enzyme-cleavable linkage with the antigen, antibody, or oligonucleotide. Reversibly quenched embodiments, in which a cleavable linkage tethers a fluorescent phthalocyanine monomer to another phthalocyanine, a heavy metal, an atom with an atomic weight greater than 80, or a paramagnetic species, are also provided for use as reporting groups in such assays.

The fluorescent phthalocyanines when monomerically tethered to monoclonal antibodies provide reagents with particular advantages for use in fluorescence microscopy, flow cytometry, and photodynamic therapy.

## Brief Description of the Drawings

FIGURE 1A presents the fluorescence emission spectrum of aluminum phthalocyanine trisulfonate in water;

FIGURE 1B presents the visible absorption spectra of aluminum phthalocyanine trisulfonate in water (plot a) as compared with aluminum phthalocyanine in dimethylsulfoxide (plot b) and in water (plot c);

FIGURES 2 and 3 depict representative embodiments of the auxiliary dipstick as disclosed in Example 8;

FIGURES 4 through 8 depict representative embodiments of the competitive binding dipstick as disclosed in Example 9; and,

FIGURES 9 through 12 depict alternative embodiments of the immunoassay device disclosed in Example 9.

## Detailed Description of the Preferred Embodiment

In one aspect, the invention provides an improved fluorescent reagent for use in analytical procedures such as immunoassays, cDNA assays, and cell sorting by which the presence and typically the quantity of some chemical, biochemical, or biological analyte is determined, often in a physiological fluid like blood plasma or urine. An ideal fluorophore for such assays would have a high fluorescence quantum yield, a large Stokes shift (>50 nm), and an emission at wavelengths greater than 600 nm. A high quantum efficiency ensures that the excitation light employed in the analytical procedure is converted efficiently into detectable emission. A large Stokes shift permits discrimination between actual signal and contaminating signal derived from Raman, Rayleigh, and Tyndall light scatter. Emission at wavelengths greater than 600 nm eliminates background fluorescence (typically from about 350 to about 600 nm) attributable to endogenous fluorophores (e.g., serum proteins, bilirubin, NADH) present in physiological fluids and to reagent and cuvette impurities.

By way of example, the following Table compares the excitation wavelength (EX), emission wavelength (EM), Stokes shift, and quantum yield (QY) of four fluorophores that have been used in such analytical procedures: 6,6'-dihydroxy-(1,1'-diphenyl)-3,3'-diacetic acid (DBDA); 4-methylumbelliferone (MUN); fluorescein (F); and rhodamine B (R-B).

| Fluorophore | EX | EM | Stokes Shift | QY |
|---|---|---|---|---|
| DBDA | 320 nm | 410 nm | 90 nm | --- |
| MUN | 360 nm | 448 nm | 88 nm | 0.5 |
| F | 495 nm | 525 nm | 30 nm | 0.5 |
| R-B | 596 nm | 620 nm | 24 nm | 0.6 |

Referring to the Table, DBDA and MUN lack optimal fluorescence properties for use in physiological assays, as both emit at wavelengths less than 500 nm. Fluorescein also emits at well below 600 nm, and furthermore has a Stokes Shift less of than 50 nm. Rhodamine B emits at greater than 600 nm but has a Stokes shift less than 50 nm. Use of a fluorophore with improved spectral characteristics would make existing assay systems more sensitive.

The invention provides such improved fluorescent reagents in the form of water-soluble, monomerically tetherable phthalocyanine derivatives according to formula 1

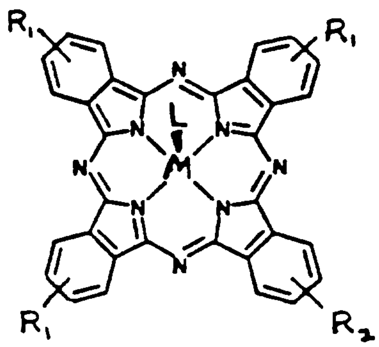

(1)

wherein, as described below, M indicates two hydrogen atoms or a metal atom selected from among aluminum, silicon, phosphorous, gallium, germanium, cadmium, magnesium, tin, and zinc; $R_1$ indicates substituents that provide water solubility; substituent $R_2$ may likewise enhance water solubility and provides a tethering linkage suitable for conjugation to another reagent moiety, or is a cleaved residue of such a linkage; and L is an axial ligand.

As a family of chromophores the metallophthalocyanines are characterized by a very narrow and strong absorption in the red envelope centered around 670 nm ($E_o = 230,000$) and a broad and less intense absorption ($E_o = 60,000$) at around 350 nm. Referring to FIGURE 1, the emission wavelength (680 nm) of the trisulfonate derivative of aluminum phthalocyanine 1, elicited by excitation at 350 nm, is red-shifted from the emissions of endogenous fluorophores in physiological solutions. A major advantage of aluminum phthalocyanine is its Stokes shift of 330 nm, which allows one to approach theoretical detection limits by the reduction of background levels. Fluorescence measurements indicate that aluminum phthalocyanine trisulfonate 1 is detectable at concentrations as low as $10^{-15}$ M, and theoretical calculations indicate a detection limit of approximately 1 x $10^{-16}$ M.

Since aluminum phthalocyanine compounds 1 possess spectral properties superior to those observed for DBDA, MUN, F, and R-B, use of an aluminum phthalocyanine species will potentially provide more sensitive assays than those employing the aforementioned species. However, to be an effective probe in biological systems, the aluminum phthalocyanine derivative 1 must be rendered soluble and disaggregated in aqueous environments and furthermore must be conjugated in monomeric form in the reagent.

The following "R" substituents ($R_1$ and/or $R_2$) bound to the phthalocyanine macrocycle 1 can serve as suitable water-solubilizing moieties: sulfonic acid groups ($-SO_3H$), sulfonate groups ($-SO_3^-$, $X^+$), carboxylic acid groups ($-CO_2H$), carboxylate groups ($-CO_2^-$, $X^+$), phosphoric acid groups ($-PO_4H_2$), phosphate groups ($-PO_4^{--}$, $2X^+$), phosphonate groups ($-PO_3H$ or $-PO_3^-$, $X^+$), hydroxy or phenoxy groups (-OH), amino groups ($-NH_2$), and ammonium and pyridinium groups ($-NR_4^+$, $X^-$). The greater the number of the R groups on derivative 1, the greater the resultant water solubility. In particular, sulfonate groups $R_1$,$R_2$ render the

compound 1 soluble over a wide range of pH's (pH = 2-12).

In the following discussion, the water-soluble and highly emissive aluminum phthalocyanine tri- and tetrasulfonates are presented as representative models for the subject group of fluorescent water-soluble metallated phthalocyanine derivatives 1. As described below in Example 1, the sulfonated aluminum phthalocyanines can be prepared by sulfonation of aluminum phthalocyanine, by metallation of free base phthalocyanine, or by total synthesis. The tri- and tetrasulfonated derivatives can serve as water-soluble precursors for preparing many of the other derivatives 1.

Because of the hydrophobic nature of the phthalocyanine macrocycle, most water-soluble phthalocyanine species exhibit a strong tendency toward aggregation. For example, the parent aluminum phthalocyanine compound 1 (wherein $R_1$ and $R_2$ are H and L is -OH) is nicely monomeric in solvents like pyridine, dimethylformamide (DMF), dimethylsulfoxide (DMSO), and methanol but is very highly aggregated and poorly soluble in water. In contrast, aluminum phthalocyanine trisulfonate suffers little aggregation in aqueous solution. Referring to FIGURE 1B, the visible absorption spectra of the parent aluminum phthalocyanine in DMSO (plot b) and in water (c) are compared to the trisulfonated derivative 1 in water (a). The close confluence of plots a and b indicates that the subject derivative 1 is essentially monomeric in aqueous solution. Two factors are considered to be responsible. First, aluminum phthalocyanine trisulfonate is highly charged in aqueous solution, which tends to electrostatically inhibit self-association. Second, the aluminum atom in aluminum phthalocyanine trisulfonate bears an axial ligand "L" (probably -OH in water) which prevents the "plate-like" stacking so prevalent with metallo(II)phthalocyanines and also porphyrins. Furthermore, as a result of aluminum phthalocyanine trisulfonate's high water solubility and relatively low hydrophobicity, its nonspecific binding to plastic, metal, and cellulose surfaces is low.

The effect of the axial ligand (L) on solubility is indicated by the observation that hydroxyaluminum phthalocyanine but not chloraluminum phthalocyanine is soluble in polar, protic solvents such as methanol. To enhance water solubility, L should be -OZ or $-NZ_2$ wherein Z is selected from among hydrogen, alkyl, acyl, and silyl groups, the latter three preferably being charged.

The quantum yield of aluminum phthalocyanine trisulfonate is approximately 0.6 in its monomeric state but diminishes to less than 4% (0.02) of its original value when aggregated. The monomeric versus dimeric (or more highly aggregated) nature of the aluminum phthalocyanine reagent is thus critical to the fluorescence emissivity and hence the signal strength and sensitivity of the assay.

To recapitulate, the high quantum yield and monomeric nature of molecule 1 are partly attributable to the aforementioned R groups and also to the central aluminum atom, which takes on an axial ligand that serves to enhance solubility and hinder the onset of aggregation. Furthermore, the central aluminum exhibits no notable heavy atom effect, which has been shown in other molecules to decrease fluorescence. It is contemplated that other metal atoms (M) with +3 valences or higher may take on an axial ligand(s) in the phthalocyanine macrocycle, thereby conferring a similar resistance to aggregation in aqueous solution. For use as fluorescent species, the metal should be diamagnetic and have a lower atomic weight than that of bromine, so as to avoid a heavy-atom quenching effect; suitable metal atoms for the latter purpose include aluminum, silicon, phosphorus, gallium, germanium, and tin. As a comparative example, the fluorescence quantum yield of copper(II)phthalocyanine trisulfonate is more than 200 times less than that of aluminum(III)phthalocyanine trisulfonate; this differential arises from aggregation and an enhancement of spin orbital coupling due to the paramagnetism of copper. Both processes give rise to radiationless deactivation of the excited state and serve to decrease the fluorescent quantum yield.

For commercial applications, the aluminum phthalocyanine species should be both chemically and photochemically stable. Phthalocyanines are an extremely stable class of chemical compounds, and aluminum phthalocyanine is particularly so. The phthalocyanines are thermally stable, and are often purified by sublimation at temperatures greater than 400 °C. Chemically, the phthalocyanines are resistant to acidic or basic hydrolysis, oxidation, and reduction. In contrast to some metallophthalocyanines, aluminum(III)-phthalocyanine is not subject to a change in oxidation state and shows no measurable tendency for demetallation. Aluminum phthalocyanine trisulfonate is also photochemically inert; with its high quantum yield of fluorescence, nearly all of its excited state energy is dissipated by emission. In addition, a study of the relative absorbance and fluorescence behavior of the sulfonated analog of aluminum phthalocyanine showed invariant physical properties over a wide range of pH conditions (pH = 4-13). Linear dynamic range studies indicated a working range of over nine orders of magnitude and superior detection limits when compared to fluorescein and rhodamine B.

Aluminum phthalocyanine and related compounds can be readily prepared from commercially available materials. The parent compound (as aluminum phthalocyanine chloride) is available from Kodak, VWR Scientific, and Strem Chemical Company. The free base phthalocyanine tetrasulfonate can be purchased from Porphyrin Products, P.O. Box 31, Logan, UT 84321. The precursors for total synthesis may be

purchased from a wide variety of sources, including Aldrich, Kodak, and Tokyo Chemical Industry Company (Tokyo Kasei Incorporated), Portland, Oregon.

Aluminum phthalocyanine derivatives that are monomeric in aqueous media can be prepared by selection of the type and number of the macrocycle functional groups R. For example, the introduction of polar or highly charged functional groups such as sulfonates tends to make aluminum phthalocyanine more water soluble and less aggregated than the corresponding carboxylated or hydroxylated species. Generally, the greater the number of polar functional groups ($R_1$ and $R_2$), the less the aggregation of the corresponding aluminum phthalocyanine derivative. For example, a tri- or tetrasulfonated aluminum phthalocyanine is more water soluble and shows less tendency toward aggregation than the corresponding mono- or disulfonated species. The more homogeneous the derivative, the greater the tendency toward aggregation. For example, a single isomer of aluminum phthalocyanine tetrasulfonate will be more aggregated than a mixture of the four isomeric tetrasulfonate derivatives.

The aluminum phthalocyanine core 1 can be readily and reproducibly functionalized to yield a water-soluble, reactive species for conjugation to proteins and other reagent moieties. For this purpose, sulfonamide, amide, ether, and thioether linkages are preferred. In other words, substituent $R_2$ can bear any amino, carboxy, thiol, or hydroxy functionality; or $R_2$ can bear the tethered analyte or other reagent moiety directly. To assure that the monomeric aluminum phthalocyanine species 1 remain monomeric, the reagent moieties should be conjugated in a medium that contains disaggregating organic solvents such as DMF or DMSO. For conjugation to proteins, such organic solvents should make up less than about 10 percent, and preferably about 5 percent, of the medium; for conjugation to smaller organic molecules, more than 10 percent and up to 100 percent of the medium should be organic solvent(s). Representative synthesis protocols for tethering sulfonated analogs of aluminum phthalocyanine are disclosed in Example 2.

Representative moieties to which such a reactive aluminum phthalocyanine can be conjugated include small physiological analytes such as drugs (therapeutic and abused), drug metabolites (e.g., cotinine), hormones, peptides, nucleotides, neurotransmitters, cholesterol, etc. A representative synthesis is presented in Example 3. Exemplary intermediate-size physiological analytes include hormones (e.g., thyroid-stimulating hormone), growth factors (e.g., nerve growth factor), oligonucleotides (cDNA, DNA, RNA, and synthetic oligonucleotide fragments), and peptides. The subject phthalocyanines can likewise be conjugated monomerically to larger reagent moieties such as antibodies, antigen-binding fragments, serum proteins, enzymes, polynucleotides (DNA, RNA), intracellular organelles, cell surface antigens, etc.

In use, the subject monomeric phthalocyanine reagents, due to their superior fluorescence properties, can be advantageously employed as detectable markers in immunoassays. Representative protocols are set forth in Examples 8 and 9. Notably, its large Stokes shift makes aluminum phthalocyanine an extremely useful reporting group for use in immunoassays (and also cDNA probe assays) for the following reasons. By way of comparison, heterogeneous immunoassays using fluorescein are limited in their sensitivity by high background interference due to the scattering of light and Raman effects. The large Stokes shift possessed by aluminum phthalocyanine substantially reduces this problem, and so assays using aluminum phthalocyanine can quantitate analytes in extremely low concentrations. Furthermore, fluorescein emits at the same wavelength as many of the fluorescent constituents of blood. Thus, in fluorescein-based immunoassays one must mechanically or by signal processing eliminate the natural background fluorescence. In contrast, aluminum phthalocyanine emits light at wavelengths beyond the range of the fluorescent emissions of blood components. Sensitive assays using aluminum phthalocyanine can thus be performed in the presence of blood with little background interference.

Spectroscopic data indicates that aluminum phthalocyanine trisulfonate has a fluorescence emission lifetime of 5 ns and a polarization coefficient of 0.005, from which we conclude that compound 1 is also suitable for a homogeneously based assay using fluorescence polarization changes upon antigen-antibody interaction when the aluminum phthalocyanine trisulfonate is tethered to a small antigen like a drug metabolite.

The ultimate sensitivity of a fluorogenic enzyme assay is determined by the spectral properties of the generated fluorophore. Water-soluble monomeric aluminum phthalocyanine species display spectral properties superior to currently available fluorophores (e.g., DBDA, MUN, F, R-B). As previously stated, aluminum phthalocyanine trisulfonate has a high quantum efficiency of emission (QY = 0.6), a large Stokes shift (335 nm), and an emission wavelength (680 nm) red-shifted from the emissions of endogenous fluorophores. Therefore, the use of an enzyme-substrate couple that enzymatically generates a monomeric aluminum phthalocyanine in the test solution would represent an improvement over the present technology.

One way to use aluminum phthalocyanine as an enzyme substrate would be to convert it to a nonemissive species that becomes emissive upon enzyme cleavage. Fluorescent molecules may be rendered nonemissive (quenched) by covalent attachment to small molecules containing heavy atoms such

as iodine. For example, fluorescein isothiocyanate (FITC) becomes less emissive upon covalent attachment to thyroxine; the iodines bound to the aromatic rings of thyroxine decrease the fluorescence emission of the fluorescein by a factor of ten. Phthalocyanines may be quenched by the presence of heavy atoms. The covalent attachment of an iodine-containing small molecule renders an aluminum phthalocyanine derivative essentially nonemissive. Representative protocols are set forth in Example 4 for the synthesis of such nonemissive aluminum phthalocyanine enzyme substrates. Once cleaved by the enzyme, the monomeric phthalocyanine is fully emissive in solution. Such reagents can be utilized in conventional enzymatic assays, e.g., immunoassays or cDNA probe assays, and as reporting groups as disclosed in Example 4.

For fluorescence microscopy or flow cytometry applications, a water-soluble monomeric form of aluminum phthalocyanine is likewise required. Water solubility is provided by derivatizing the aluminum phthalocyanine with moieties such as sulfonic acid ($SO_3H$) as described above. The water-soluble aluminum phthalocyanine is derivatized to provide a reactive functionality for attachment to proteins such as antibodies or avidin. For example, a water-soluble aluminum phthalocyanine can be activated for such attachment as described in Example 5.

Aluminum phthalocyanine possesses several properties that make it particularly advantageous as a label for flow cytometry. The absorption maxima at 350 nm means that the aluminum phthalocyanine reagent can be efficiently excited by the argon lasers that are currently widely used in flow cytometry, and also by the newer, inexpensive helium-cadmium and helium-neon lasers. The emission at 680 nm means that with the aluminum phthalocyanine reagents there is no spectral overlap of emission with existing dyes, no interference due to Raman, Rayleigh, or Tyndall light scatter, and no auto or background fluorescence. Aluminum phthalocyanine-antibody conjugates present several unique opportunities for multiparameter analysis. First, the simultaneous use of aluminum phthalocyanine, fluorescein, and phycoerythrin for the first time provides a method for performing three-parameter cell surface analysis using a single argon laser. Second, aluminum phthalocyanine-antibody conjugates and the DNA stain Hoechst 33342 can be used with a helium-cadmium laser to perform simultaneous cell-surface and cell-cycle analyses.

In another aspect, the invention provides chromogenic monomeric phthalocyanine reagents for use in colorimetric immunoassays. For such applications the phthalocyanine 1 is preferably metallated with copper, but other metals (M), notably aluminum, or silicon, can be used. Because aggregation reduces the molar absorptivity of the phthalocyanine species, any of the foregoing strategies that decrease aggregation (such as the provision of an axial ligand) can be employed to provide a more absorptive species. Example 6 sets forth representative protocols for the preparation of such reagents.

In a further aspect, the invention provides therapeutic monomeric phthalocyanine reagents. For photoimmunotherapy, it is critical that the photosensitive agent retain its photodynamic activity after conjugation to a tumor-directed antibody or binding fragment. This criterion is satisfied in the case of metallophthalocyanine sensitizers by ensuring that the phthalocyanine reagents are monomerically coupled to the antibody, for self-quenching of the aggregated species would otherwise impair the generation of singlet oxygen in vivo. Preferred metals (M) for this purpose are aluminum and zinc. Suitable protocols for monomerically binding the phthalocyanine to an antibody are presented in the Examples.

The invention is further illustrated by the following representative Examples.

## EXAMPLE 1

### Preparation of Sulfonated Aluminum Phthalocyanine Derivatives

There are several reported methods for the synthesis of sulfonated phthalocyanine compounds. As examples, one may treat a phthalocyanine with: fuming sulfuric acid; sulfuric acid monohydrate with 20% oleum; chlorosulfonic acid followed by hydrolysis; or sulfur trioxide-pyridine. Sulfonation of aluminum phthalocyanine (AlPc) with oleum yields a mixture of aluminum phthalocyanine compounds containing predominantly the trisulfonate. Representative synthesis protocols follow.

Chlorosulfonic acid method: In a representative synthesis, 3.0 mL chlorosulfonic acid was added to 396 mg (.069 mmol) chloro-aluminum phthalocyanine in a 25 mL round bottom flask fitted with a stir bar. The mixture was stirred to effect dissolution, sealed under argon, and immersed in a preequilibrated oil bath at 145 °C. The solution was stirred at 145 °C for 2 hours, cooled to 0 °C, and quenched by gradual addition to 25 g of ice. The solid contained within the resultant slurry was collected by suction filtration, washed with 100 mL $H_2O$, 300 mL $CH_2Cl_2$, and then dried under partial vacuum for several hours. The resultant blue solid was characterized by elemental analysis and found to have a N:S ratio consistent with that anticipated for the aluminum phthalocyanine trisulfonyl chloride. The $^1H$ NMR (500 MHz) analysis reveals that the material is a mixture of sulfonated materials. The temperature and time utilized for sulfonation greatly affect

the product distribution.

Conversion to aluminum phthalocyanine trisulfonate: To 200 mg of predominantly aluminum phthalocyanine trisulfonyl chloride was added 1.0 mL 1N NaOH. This mixture was stirred for 24 hours at room temperature, concentrated under vacuum, and purified by preparative TLC ( 1 part 10% $NH_4OH$ to 3 parts methanol). The resultant blue solid was analyzed by elemental analysis, and found to have a N:S ratio consistent with aluminum phthalocyanine trisulfonate. Analysis by $^1H$ NMR reveals that this material is a mixture of sulfonated materials.

The sulfonations of aluminum phthalocyanine described above result in mixtures of phthalocyanines, in which the average number of sulfonate groups per phthalocyanine is three.

Metallation of free base phthalocyanine tetrasulfonate: Free base phthalocyanines, as with the porphyrins, can be metallated with a variety of species to yield the corresponding metallo analogs. Metallation may be effected with a variety of reactive metal sources including trimethyl metal and metal trictuoride. For example, free base tetraphenylporphine has been metallated with $(CH_3)_3Al$ to yield the corresponding aluminum derivative. To date, the metallation of a sulfonated free base phthalocyanine with a reactive derivative of aluminum has not been reported. Thus, the following novel synthesis is presented.

To a dry 10 mL round bottom flask fitted with stir bar was added 50 mg (0.06 mmol) free base phthalocyanine tetrasulfonate. The flask was sealed under argon, and the contents were diluted with 1.0 mL dimethylformamide (DMF). After stirring 10 minutes at room temperature to effect dissolution, the solution was treated with 0.7 mL (1.3 mmol) $(CH_3)_3Al$ (1M in toluene), stirred at 25°C for 12 h, and concentrated under vacuum. Extraction with methanol followed by concentration of the methanolic solution under vacuum led to the isolation of 5 mg methylaluminum phthalocyanine.

Total synthesis: Metallophthalocyanines can be prepared by reaction of: phthalonitriles with metal salts (phthalonitrile process); phthalic acid derivatives with urea and metal salts (urea process); or diiminoisoindoline derivatives with metal salts. See: The Phthalocyanines, Volume 2, F.H. Moser and A.L. Thomas, CRC Press, Boca Raton, FL, 1983. A total synthesis of true tetrasulfonated aluminum phthalocyanine has not been reported. The urea method is described in Weber, J.H. and D.H. Busch, *Inorg. Chem. 4*:469, 1965; Bauman, F., U.S. Patent No. 2,613,128; and Fukada, N., *Nippon Kagaku Zasshi, 75*:1141, 1954. The products of such reactions are necessarily a mixture of the four isomers of tetrasulfonated aluminum phthalocyanine. Exemplary total syntheses of aluminum phthalocyanine tetrasulfonate follow:

Synthesis via melt: A finely ground mixture of 133 mg (1.0 mm) aluminum trictuoride, 1.25 g (4.0 mm) 4-sulphophthalic acid trisodium salt, 1.20 g (20 mm) urea, 107 mg (2.0 mm) ammonium chloride, and 15 mg (0.012 mm) ammonium molybdate was heated at 280°C under nitrogen for 2.5 hours. After cooling, the crude product was extracted with 50 ml methanol and concentrated. The product was taken up in 20 ml distilled water and dialyzed exhaustively against distilled water (3,500 MW cutoff dialysis tubing). The aluminum phthalocyanine tetrasulfonate was produced in about 10% yield and was characterized by 1H NMR, UV/Vis absorbance, and fluorescence spectroscopies.

Synthesis via solution: Heating a solution of the above reactants in 10 ml nitrobenzene at 180°C for 4 hours under nitrogen also produced aluminum phthalocyanine tetrasulfonate in approximately the same yield. Nitrobenzene was decanted from the crude reaction mixture and the residue was washed with benzene to remove the remaining traces of nitrobenzene. The solid was taken up in distilled water and purified by dialysis as above.

The starting material for the above preparations is sulfophthalic acid which is commercially available for Aldrich Chemical Company as the trisodium salt and from Tokyo Chemical Industry Company (TCI) as the triammonium salt. Both products are a mixture of 3-sulfo and 4-sulfophthalic acid. The Aldrich material is a 3:1 mixture with 4-sulfo predominating; TCI's product is approximately 1:1. The number of isomeric tetrasulfonated phthalocyanines produced from these starting materials is therefore greater than four.

EXAMPLE 2

Preparation of Reactive Aluminum Phthalocyanine Derivatives

Representative synthesis protocols for tethering sulfonated analogs of aluminum phthalocyanine are disclosed below.

Direct attachment via reactive sulfonic acid derivative: A reactive aluminum phthalocyanine sulfonic acid derivative can be covalently attached directly to any physiological analyte (A) that contains a reactive nucleophile (e.g., $A-NH_2$, A-OH, A-SH, etc.) For example, any amino compound can be coupled to aluminum phthalocyanine trisulfonyl chloride. The following representative protocol describes such a coupling to *p*-amino benzoic acid (PABA). To a stirred solution of 42 mg (0.4 mmol) $Na_2CO_3$ in 1.0 mL $H_2O$

at 80 °C was added 27 mg (0.2 mmol) PABA. After stirring 5 minutes at 80 °C, 100 mg (0.1 mmol) aluminum phthalocyanine trisulfonyl chloride was added gradually with stirring. After 12 hours at 80 °C, the mixture was cooled to 25 °C and then concentrated under vacuum. The resultant blue solid was subjected to exhaustive acetone trituration to yield the corresponding mono-PABA-sulfonamide.

Alternatively, reagent moieties can be coupled to a water-soluble aluminum phthalocyanine derivative by activation of the sulfonic acid residues, as in the following protocol for attaching PABA to aluminum phthalocyanine trisulfonate. To a 10 mL round bottom flask containing a stirred solution of 150 mg (0.17 mmol) aluminum phthalocyanine trisulfonate in 2.0 mL benzene at 25 °C was added dropwise 0.75 mL (8.6 mmol) oxalyl chloride. After 6 hours at room temperature, the solvent was evaporated in vacuo to yield aluminum phthalocyanine trisulfonyl chloride as a dark blue solid. To a stirred solution of 61 mg (0.58 mmol) of $Na_2CO_3$ in 1.0 mL $H_2O$ at 80 °C, was added 31 mg (0.23 mmol) PABA. After 5 minutes at 80 °C, 55 mg (0.06 mmol) of the aluminum phthalocyanine trisulfonyl chloride was added. The mixture was stirred at 80 °C for 6 hours, and the solvent was removed by concentration under vacuum. The contents of the flask were diluted with 10% $NH_4OH$ in methanol, reconcentrated under vacuum, and then exhaustively triturated with acetone to give mono-PABA-sulfonyl aluminum phthalocyanine disulfonate.

Attachment of analytes via an amino aluminum phthalocyanine derivative:Sulfonated aluminum phthalocyanine can be converted to amino derivatives by reaction of the corresponding sulfonyl chloride with a diamino compound. For example, to a solution of 24 mg $Na_2CO_3$ in 1.0 mL $H_2O$ at 80 °C was added 15 mg 2,2'-oxybis(ethylamine)hydrochloride. After 5 minutes at 80 °C, 50 mg aluminum phthalocyanine sulfonyl chloride was added, the mixture was diluted with an additional 0.5 mL of $H_2O$, and heated at 80 °C for 12 hours to yield the corresponding monoamino derivative.

Attachment of analytes via carboxy aluminum phthalocyanine derivative: Activation of a sulfonated aluminum phthalocyanine followed by treatment with an amino acid yields a mono-, di-, tri- or tetra-carboxy functionalized sulfonated aluminum phthalocyanine. The resultant species is activated to a mixed anhydride by treatment with ethyl chloroformate in triethylamine and dimethylformamide, or coupled directly to reactive nucleophiles ($A-NH_2$, etc.) on the analyte. An exemplary synthesis is presented in Example 3.

EXAMPLE 3

Preparation of Aluminum Phthalocyanine-Morphine Conjugate

To a stirred solution of 50 mg $(PABA)_1-(SO_2)-AlPc-(SO_2Cl)_2$ in 0.5 mL triethylamine at 0 °C was added 7 µl ethylchloroformate. After 5 minutes at 0 °C, 29 mg 3-(4-aminobutyl)morphine was added; the reaction mixture was warmed to room temperature and stirred for 8 hours to yield the corresponding monomorphine functionalized derivative.

The aluminum phthalocyanine-morphine conjugate described above was evaluated spectroscopically by UV-VIS and fluorescence techniques. These data indicate that the conjugated morphine analog was 76% as emissive as the parent (quantum yield = 0.46), and that direct monomeric conjugation of the aluminum phthalocyanine molecule to a small hapten has very little influence on the emission yield.

The relative immunoaffinity of morphine, the amino morphine analog, and the amino-morphine derivative of mono-PABA-sulfonyl aluminum phthalocyanine disulfonate was determined in a competition experiment with antimorphine monoclonal antibody and tritium-labeled morphine. The relative affinity of the antibody for each of these species is shown in the following table.

| Competitor | Relative Affinity |
|---|---|
| morphine | 1 |
| amino morphine | 1 |
| aluminum phthalocyanine-morphine | ~ 1 |

These results indicate that functionalization of morphine to yield a tetherable analog does not disrupt antibody recognition, and that attachment of approximately one morphine analog to an aluminum phthalocyanine derivative yields a species that is as competitive as the parent compound in an immunoassay.

EXAMPLE 4

Preparation of Reversibly Nonemissive Aluminum Phthalocyanine Reagents

An improved fluorogenic assay is configured as a heterogeneous competitive enzyme assay in which the analyte and an analyte-enzyme conjugate are incubated with an analyte-specific antibody that recognizes both the analyte and the analyte-enzyme conjugate. Competition ensues for binding sites on the antibody. As the amount of analyte present in the test solution increases, the amount of analyte-enzyme conjugate bound to the antibody decreases. After an incubation period, the antibody-bound analyte and analyte-enzyme conjugate reagents are removed from solution, for example, by adding solid-phase immobilized antibody directed against the analyte-specific antibody. The antibody-bound analytes and analyte-enzyme conjugates are thereby immobilized on the solid phase, which is then washed to remove all traces of free analyte and analyte-enzyme conjugate. Thereafter, the solid phase is incubated with a nonemissive aluminum phthalocyanine conjugate that can be enzymatically cleaved to yield a highly emissive monomeric aluminum phthalocyanine derivative in the test solution. If the solid phase contains analyte-enzyme conjugate, monomeric phthalocyanine derivative will be generated and the resultant fluorescence signal will be indirectly proportional to the amount of analyte that was present in the test solution.

The nonemissive aluminum phthalocyanine derivative that becomes emissive upon enzyme cleavage can be configured in several ways that take advantage of the heavy atom effect, dimerization (self-quenching), or charge transfer.

Heavy atoms such as iodine are known to quench the fluorescence emission of phthalocyanines. Thus, the reagent conjugate for this assay can take the form of an aluminum phthalocyanine derivative with an iodine atom covalently attached via an enzyme-cleavable linkage. The iodine atom may be so linked to the aluminum phthalocyanine derivative at the axial position (L) or to the macrocycle or substituents (R) thereon. Since horseradish peroxidase is known to cleave aromatic and aliphatic iodides, representative species 2-4 (wherein "$-CH_2I$" here indicates any tether that bears an alkyl or acyl iodide) can function as horseradish peroxidase substrates.

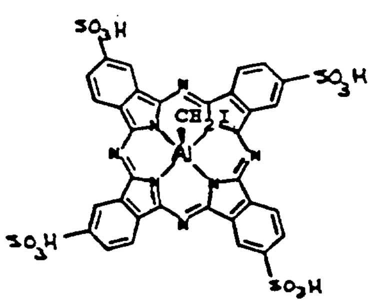

(2)

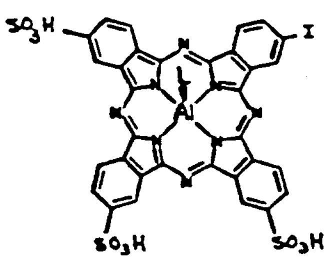

(3)

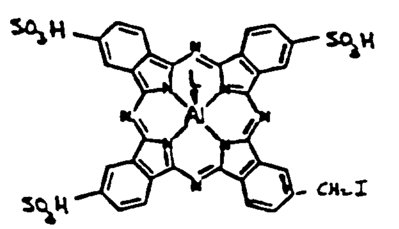

(4)

Alternately, the heavy metal atom or a paramagnetic species (e.g., a copper or iron atom, a nitroxide, or other spin label) is attached to the aluminum phthalocyanine derivative by any of a number of enzyme cleavable linkages. For example, attachment of the iodine via a galactose residue, as illustrated in representative species 5 and 6, yields a $\beta$-galactosidase enzyme substrate.

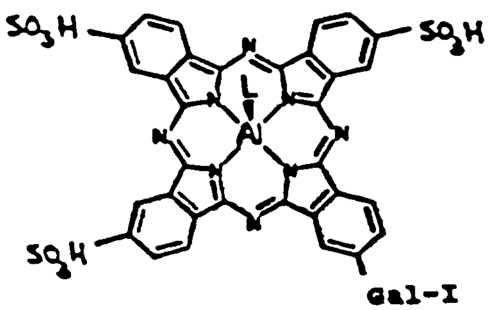

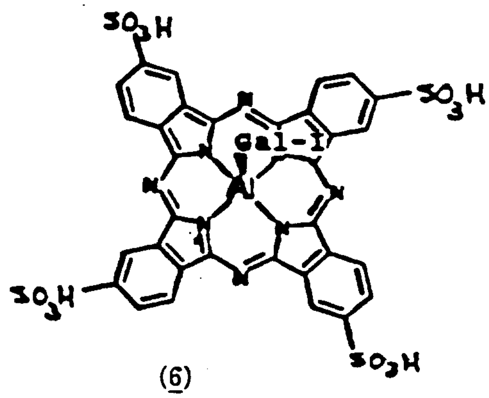

(5) (6)

Covalent dimers of aluminum phthalocyanine may alternatively be generated by axial ligation or macrocycle dimerization. Three exemplary embodiments 7, 8a, and 8b are illustrated, in which enzyme-cleavable linkage(s) (Ec) serve to reversibly bind the monomers to one another.

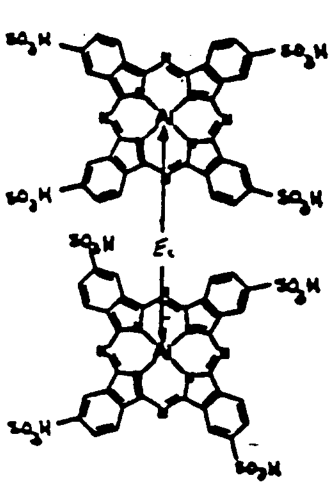

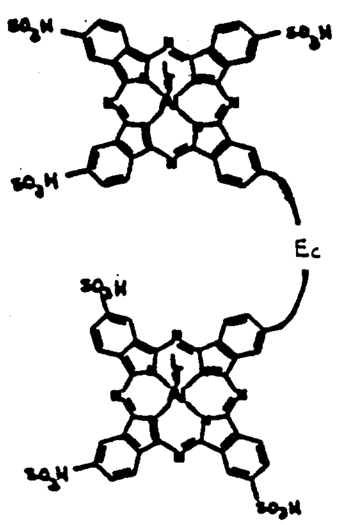

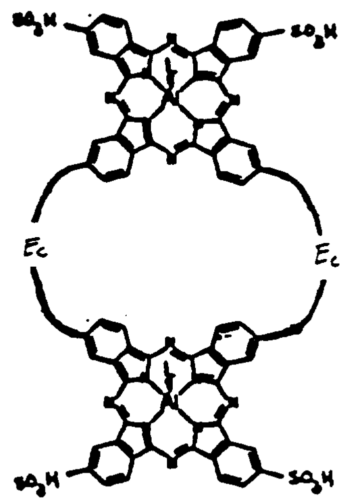

(7) (8a) (8b)

For example, the two species may be joined by a galactose moiety thus providing a $\beta$-galactosidase substrate.

EXAMPLE 5

Monomeric Aluminum Phthalocyanines in Fluorescence Microscopy and Flow Cytometry

Direct conjugation of aluminum phthalocyanine to a protein molecule such as bovine serum albumin (BSA) or avidin was found to yield a protein conjugate with unexpectedly low emissivity. As a result, we have determined that the most highly emissive aluminum phthalocyanine protein conjugates will result from indirect coupling with the use of an intermediate tether. Such a linking tether should be at least two and preferably from about four to about twelve atoms in length. Synthetic protocols employing two different types of tethers are outlined below.

In a first protocol, treatment of aluminum phthalocyanine (AlPc) with chlorosulfonic acid followed by aqueous quench and isolation leads to the formation of aluminum phthalocyanine trisulfonyl chloride. Reaction of the trisulfonyl derivative and any of a variety of amino acids in an organic solvent yields a

carboxylic acid functionalized derivative. Activation of the carboxylic acid functionality followed by direct coupling of the activated derivative to the amino groups of either streptavidin or an antibody in a dissociating solution containing less than ten percent organic solvents yields the desired monomeric aluminum phthalocyanine-protein conjugate.

In a second synthetic protocol, treatment of aluminum phthalocyanine trisulfonyl chloride with any of a number of diamines leads to the formation of an amino derivative. Coupling of succinimidyl-4-(N-maleimidomethyl) cyclohexane-1-carboxylate (SMCC) to the amino derivative yields the corresponding maleimide. A separate reaction of the amino groups of either an antibody or streptavidin with S-acetylmercaptosuccinic anhydride in sodium phosphate buffer, followed by quenching and Sephadex purification, yields a thiolated species. Conjugation of the thiolated protein with the maleimide derivative in the dissociating solution gives the desired monomeric aluminum phthalocyanine-protein conjugate.

Sample Analysis: Peripheral blood lymphocytes (PBL) are separated by standard Ficoll-Hypaque gradient. Cells are labeled with either aluminum phthalocyanine-conjugated antibody or biotinylated antibody by incubation in saturating concentration of antibody at 4 °C for 30 minutes, followed by washing in phosphate-buffered saline. Biotin-labeled cells are then incubated with 10µg/ml aluminum phthalocyanine-conjugated streptavidin for 30 minutes at 4 °C, followed by washing. For two- and three-color fluorescence analyses, the cells are labeled with FITC- and phycoerythrin-conjugated antibodies simultaneously with the aluminum phthalocyanine- or biotin-conjugated antibodies. For assays that include DNA staining of viable cells, the cells are incubated with 10 µg/ml Hoechst 33342 dye at 37 °C for 30 minutes, prior to the antibody labeling at 4 °C.

Flow cytometric analysis of aluminum phthalocyanine-labeled cells can be performed with excitation from a dye laser, a helium-neon laser, or the UV lines of either an argon ion laser or a helium-cadmium laser. Emission is detected at around 680 nm. FITC and phycoerythrin can be excited with the spatially separated beam of an argon ion laser at 488 nm, with fluorescence emission detected at 520-540 nm and 563-587 nm, respectively. Hoechst dye (DNA-specific) fluorescence can be elicited by the 351-364 UV laser lines of an argon ion laser, with emission detected above 420 nm.

EXAMPLE 6

Chromogenic Monomeric Phthalocyanine Reagents

The following Example discloses representative chromogenic analyte conjugates for use in immunoassays, and protocols for their preparation. These representative analyte conjugates employ bovine serum albumin (BSA) as a macromolecular carrier. To the BSA core are attached monomeric copper phthalocyanine marker components and analyte components (theophylline, in this instance). While BSA is here described as the carrier, any similarly functionalized carrier species would suffice.

Marker component: selection and conjugation. Copper phthalocyanine 9 was selected as a potential marker component for the analyte conjugate due to its high molar absorptivity ($\lambda_{max}$ 678.5 nm, $\epsilon$ = 218,500 in chloronaphthalene). *J. Chem. Soc.*, 2466, 1957. A modified version 10, rendered both water-soluble by sulfonation and reactive by conversion to a dichlorotriazinylaminoethane sulfonamide, is readily available in crude form from several dye manufacturers (e.g., MX-G; Pro Chemical and Dye, Inc., Somerset, MA).

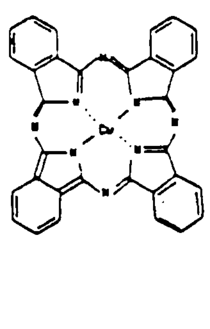

(9)

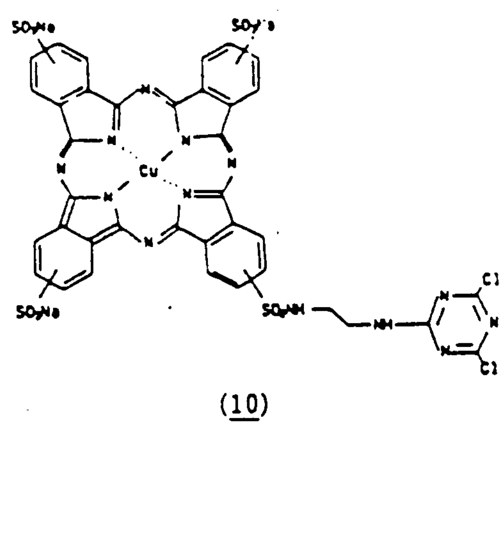

(10)

In spite of the high molar absorptivity of the parent copper phthalocyanine compound 9, we found that the dichlorotriazinylethylene diamine sulfonamide derivative 10 (hereinafter referred to as "CuPc") displayed an $\epsilon$ of 34,615 at $\lambda_{max}$ 666 nm in pH = 8 phosphate buffer. Purification by column chromatography (silica, Dowex, cellulose Sephadex) or high performance liquid chromatography (HPLC) led to only at 15% increase in $\epsilon$, suggesting that factors other than impurities were responsible for the observed decrease in molar absorptivity ($\epsilon$). Related compounds are found to aggregate to form dimers and/or higher oligomers, with a dramatic decrease in $\epsilon$, when dissolved in aqueous solution at a variety of pH's and temperatures. *Aust. J. Chem. 25*:1661-1667, 1972. The bulk of such aggregates are dimers, $(CuPc)_2$, and so are referred to hereinafter as such.

Compound 10 was linked to bovine serum albumin (BSA) under reaction conditions analogous to those described in *J. Immunol. Meth. 13*:305, 1976, for optimal binding of dichlorotriazinylaminofluorescein to IgG. To a stirred solution of BSA (25 mg, 3.6 x $10^{-4}$ mmol) in pH = 8 phosphate buffer (2.0 mL) was added compound 10 (100 mg, 3.6 x $10^{-2}$ mmol). The mixture was stirred at 25 °C for 22 hours and then filtered through Sephadex G-25 with pH = 8 phosphate buffer (10.0 mL). The filtrate was concentrated by Amicon ultrafiltration (50 K; 600 mL pH = 8 phosphate buffer, 600 mL distilled $H_2O$) and lyophilized to yield conjugate 11 (22 mg) as a blue solid with $\epsilon_{mol}$ = 400,000, assuming a MW = 80kd for conjugate 11.

BSA - $[(CuPc)_2]_{10}$ (11)

Related conjugates were prepared as shown in the following table:

| [BSA] | Eq(10)+ | [(10)] | ml Buffer | Time | Eq(10)/BSA* | Solubility |
|---|---|---|---|---|---|---|
| $2.9x10^{-5}$ | 103 | $3x10^{-3}$ | 1.0 | 1 h | 0.8 | v |
| $3.6x10^{5}$ | 8 | $3x10^{-4}$ | 10.0 | 22 h | 0.9 | v |
| $2.9x10^{-5}$ | 34 | $1x10^{-3}$ | 1.0 | 20 h | 5 | v |
| $1.8x10^{-4}$ | 100 | $2x10^{-2}$ | 10.0 | 22 h | 10 | v |
| $2.5x10^{-4}$ | 497 | $1x10^{-1}$ | 10.0 | 52 h | 19 | s |

+Equivalents of compound 10.
*Assumes 10 exists as a dimer.

Referring to the table, the number of dimers of compound 10 per BSA molecule, Eq(10)/BSA, was determined, after purification, using $\epsilon$ = 40,000 per dimer and assuming a molecular weight of 70,000. All the conjugates were highly water soluble (v), with the exception of the last entry (s) in the table. The binding of approximately 19 dimers substantially decreased the water solubility of the BSA carrier.

Ready visualization (A>0.1) of most therapeutic drug analytes in a test solution containing the dimer-linked BSA (based upon an $\epsilon$ of 40,000 per dimer unit), requires the binding of at least 8 dimers of 10 per carrier. Such a sensitivity would allow the detection of analytes such as theophylline (10-100 ng/ml blood serum) but not analytes at concentrations in test fluid below on the order of 10 ng/ml, such as digoxin (0.5-2 ng/ml). In contrast, the corresponding monomer-linked BSA (based upon conjugation with eight monomers and an $\epsilon$ of 200,000 per monomer unit; see below) would expand the lower detection limit by a factor of five ( $\epsilon$ monomer = $5\epsilon$ dimer ) to encompass analytes occurring at concentrations in the 2 to 20 ng/ml range.

Aggregated copper phthalocyanine derivatives are reported to disaggregate in aqueous solutions containing urea and thiourea (Aust. J. Chem. 26:1545, 1973) or solvents such as acetone (Aust. J. Chem. 25:1661-1667, 1972). Our attempts to either disaggregate the bound dimer from conjugates like 11 or prepare the monomeric analog of 11 were unsuccessful in urea. However, conjugation of BSA to 10 in certain acetone:buffer media led to the formation of predominantly monomeric copper phthalocyanine-linked BSA 12 with $\epsilon_{BSA}$ greater than $2x10^6$.

BSA-$(CuPc)_{10}$ (12)

The order of addition as well as the ratio of acetone:buffer proved to be very important in optimizing the coloring of the conjugates. Optimum pigmentation was achieved when the copper phthalocyanine derivative 10 was added to a solvent system composed of greater than 50 percent acetone in pH = 8 phosphate buffer prior to the gradual addition of dry BSA. A representative synthesis follows. BSA (75 mg, 1.1 x $10^{-3}$ mmol) was added gradually to a stirred solution of compound 10 (109 mg, 1.1 x $10^{-1}$ mmol) in a 70:30 mixture of acetone (87.0 mL) and pH = 8 phosphate buffer (37.0 mL). The mixture was stirred at 25 °C for 48 hours,

allowed to stand 24 hours, and then filtered through Sephadex G-25 with pH = 8 phosphate buffer (30.0 mL). The filtrate was purified and concentrated by Amicon ultrafiltration (50 K; 3.0 L pH = 8 phosphate buffer, 2.0 L distilled $H_2O$), and lyophilized to yield conjugate 12 (17 mg) as a dark blue solid with $\epsilon_{mol}$ = 2,100,000, assuming a MW of 80,000 for 12.

Monomeric phthalocyanines can be similarly linked to other amine-bearing proteins, such as antibodies and antigens, to provide colored reagents useful for immunoassays generally. Aluminum phthalocyanine derivatives, in addition to being suitable chromogens, are advantageously luminescent as well.

Ligand component: biotinylation. Marker-labeled conjugates 11 and 12 were biotinylated with N-hydroxysuccinimidyl biotin (NHS-biotin) as described in the following representative protocol. To a stirred solution of conjugate 11 (5.0 mg, 6.3 x $10^{-5}$ mmol) in pH = 7 PBS (100 $\mu$l) was added NHS-biotin (0.4 mg, 1.3 x $10^{-3}$ mmol) and a trace of $^3$N-NHS-biotin in dimethylformamide (DMF) (100 $\mu$l) and pH = 7 PBS (20 $\mu$l). The mixture was stirred at 25 ° C for two hours and then filtered through Sephadex G-25 with distilled $H_2O$ (600 $\mu$l) to yield biotinylated conjugate 13 as a clear blue solution. The extent of radiolabel incorporation indicated that each molecule of 13 was biotinylated with an average of 10 biotin molecules.

$(Biotin)_{10}$-BSA-$[(CuPc)_2]_{10}$ (13)

Preliminary experiments conducted with such highly biotinylated conjugates of 11 and 12 revealed that neither species had any avidin-binding efficiency. Using the procedure described above, we biotinylated conjugates 11 and 12 with a commercially available chain-extended biotin (sulfosuccinimydyl 6-biotinamidolhexanate; Pierce Chemical Co., Rockford, IL) to produce the analogous biotinylated species bearing 15 and 12 biotins, respectively. The resulting conjugated species were found to possess the requisite affinity for avidin.

Analyte component: tethered theophylline derivative. A tethered theophylline derivative was prepared for conjugation with protein, for use in an analyte conjugate to assay for theophylline in blood serum. First, theophylline acid 14 was prepared as described in Res. Comm. Chem. Path. Pharm. 13:497, 1976. A solution of 1.0 g (5.9 mmol) 5,6-diamino-1,3-dimethyluracil hydrate and 1.34 g (11.8 mmol) glutaric anhydride in 10.0 ml N,N-dimethylaniline was heated at 200 ° C for three hours under nitrogen. After cooling to room temperature, the crystals that formed were collected by filtration. The crystals were washed with benzene and recrystallized from water. Theophylline acid 14 was isolated as white granules, 700 mg (45%).

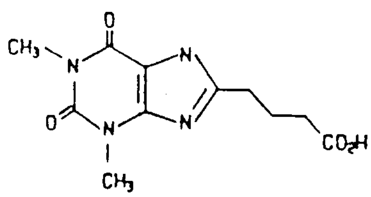

(14)

To a solution of 50 mg (0.188 mmol) acid 14, 4.0 ml pyridine, and 1.0 ml dioxane was added 43.2 mg (0.225 mmol) 1-ethyl-3-(3-dimethylaminopropyl) carbodiimide. The solution was stirred for one hour at room temperature. A solution of 35 mg (0.20 mmol) mono-t-butylcarbamate of 1,3-diamino-propane in 1.0 ml dioxane was added, and the resulting solution was stirred overnight. The reaction mixture was diluted with 10 ml methylene chloride, washed with dilute aqueous hydrochloric acid, dried over sodium sulfate and concentrated. Compound 15 was isolated as a white crystalline solid, 70 mg (88%).

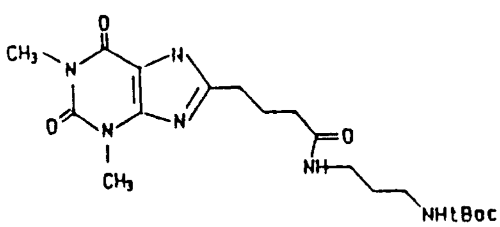

(15)

To 50 mg (0.118 mmol) carbamate 15 was added 1.0 ml ice-cold trifluoroacetic acid/methylene chloride (1:3). The solution was stirred at 0 ° C for one hour. Removal of solvent gave amine 16 as a clear, colorless

oil, 36 mg (96%).

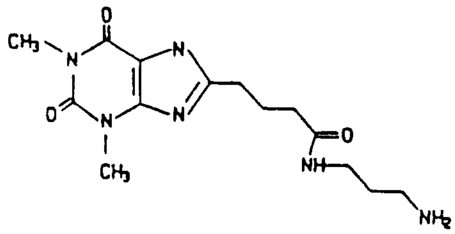

(16)

To a solution of 40.8 mg (0.118 mmol) succinimidyl 4-(N-maleimido-methyl) cyclohexane-1-carboxylate (SMCC) in 1.0 ml DMF was added 36 mg (0.112 mmol) amine 16 in 1.0 ml DMF. The resulting solution was stirred overnight at room temperature. The reaction mixture was diluted with 10 ml methylene chloride, washed with 3-10 ml portions of water, and dried over magnesium sulfate. Removal of solvent gave maleimide 17 as a crystalline solid, 38 mg (63%).

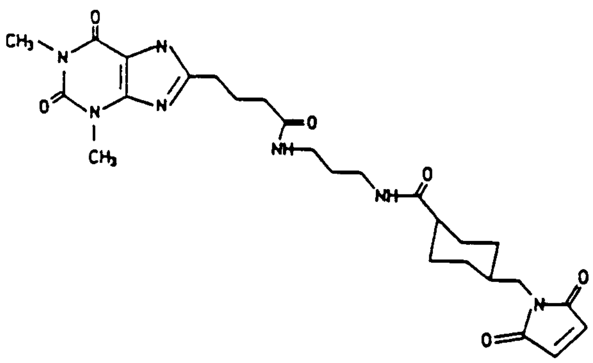

(17)

Theophylline derivative 17 is suitable for conjugation to sulfhydryl bearing species such as the BSA derivatives prepared below. Sufficient length has been built into this tether to maximize the interaction of the theophylline (analyte) component of the analyte conjugate with the antibody (analyte binding partner). To assure the recognition of the theophylline-protein conjugate by the antibody, the immunogen should mimic the analyte conjugate, that is, the tether point to theophylline should be consistent for both purposes.

Incorporation of sulfhydryl groups into BSA. To a solution of 5.0 mg ($7.58 \times 10^{-5}$ mmol) BSA in 1.0 ml PBS (pH7) was added the desired number of equivalents of N-succimidyl 3-(2-pyridyldithio)-propionate (SPDP) as a solution in absolute ethanol. After one hour at room temperature, the solution was filtered through Sephadex G-25.

To the resulting solution of the BSA/SPDP conjugate was added 1.2 mg ($7.58 \times 10^{-3}$ mmol) dithiothreitol (DTT). After 30 minutes at room temperature, the sulfhydryl (SH) content of the protein was assayed by measuring the absorbance of 2-thiopyridone at 343 nm. Representative results are shown below.

| Eq. SPDP | SH/BSA |
|---|---|
| 5 | 4 |
| 10 | 6 |
| 25 | 13 |
| 50 | 28 |

Conjugation of analyte component to BSA. The above BSA/SPDP conjugates bearing free sulfhydryl groups were reacted with theophylline analog 17 as follows. To a solution of 30 mg ($4.55 \times 10^{-4}$ mmol) BSA/SPDP conjugate (SH/BSA determined to be 3) was added 30 equivalents ($1.37 \times 10^{-2}$ mmol) theophylline maleimide 17. After stirring overnight at room temperature, the reaction mixture was filtered through Sephadex G-25 and dialyzed against distilled water. Lyophilization gave 25 mg of a

BSA/theophylline conjugate. Determination of 3 theophylline haptens per BSA was made by absorbance measurement at λ280 nm.

Construction of exemplary analyte conjugates. In the analyte conjugate, separate marker, ligand, and analyte components may be bound directly to a single carrier molecule. A representative analyte conjugate 18, shown schematically below, is preferably formed by covalently binding monomeric phthalocyanines to BSA, followed by biotinylation of the BSA and conjugation of theophyllines to the BSA, all as described above.

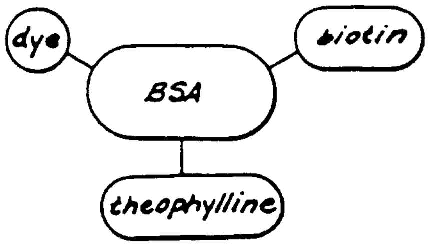


(18)

The biotin or other designated ligand component may, of course, be omitted from the analyte conjugate.

Two embodiments of the analyte conjugate which employ pairs of carrier molecules are shown below.

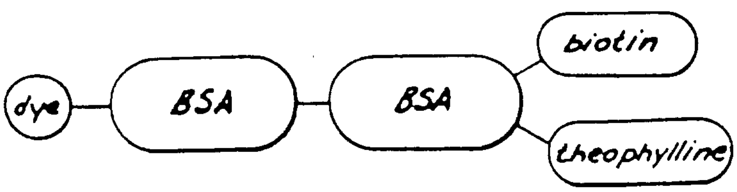


(19)

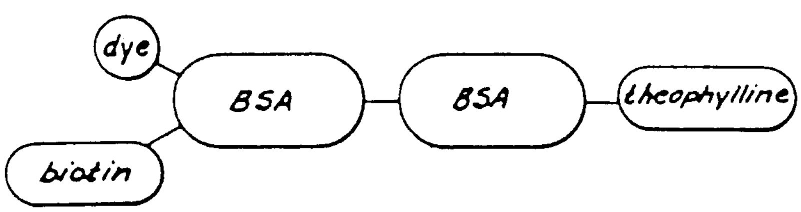


(20)

The preparation of these analyte conjugates follows the protocols described above; namely, dye binding, biotinylation, and theophylline conjugation to BSA. Formation of analyte conjugate 19 involves the linking of a BSA bearing phthalocyanine components to a BSA bearing biotins and theophyllines. Similarly, conjugate 20 is formed by linking a biotinylated, dye-labeled BSA to a BSA to which theophyllines have been conjugated. The BSA-BSA link is accomplished by introduction of sulfhydryl groups into one of the BSA components, as described above, and the incorporation of functionalities reactive toward sulfhydryls, such as alpha-iodoacetates or maleimides, into the other BSA component.

An extension of this methodology results in the modular analyte conjugate 21 represented schematically below.

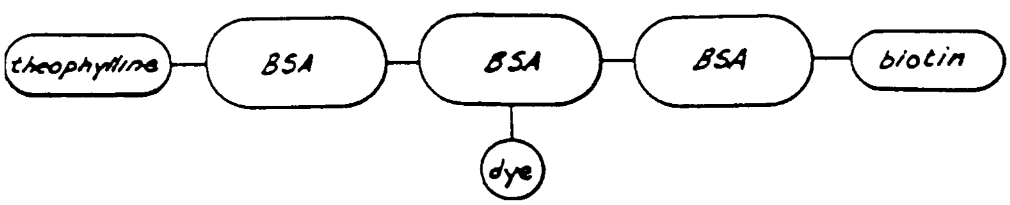


(21)

The preparation of the individual BSA conjugates and the assembly of the BSA links are as described above.

Avidinized analyte conjugate. In a complementary embodiment, the analyte conjugate may contain an avidin ligand component. Two representative avidinized analyte conjugates are shown schematically below.

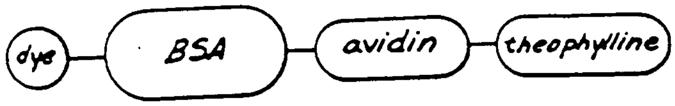


(22)

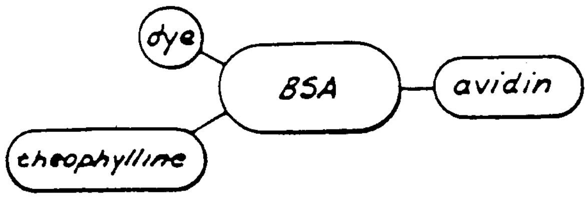


(23)

Preparation of 22 and 23 follow from the chemistries described above. For 22, a dye-BSA conjugate is covalently linked to an avidin-theophylline conjugate in a manner analogous to the linking of BSA conjugates described above. The formation of 23 involves the linking of avidins to a BSA bearing both dyes and theophyllines.

BSA protected dye-BSA conjugate. The hydrophobicity of the copper phthalocyanine components of the above analyte conjugates may lead to nonspecific binding of the conjugates to some surfaces, such as polyethylene and other plastics. This potential problem is alleviated by blanketing the dye-BSA complexes with additional BSA.

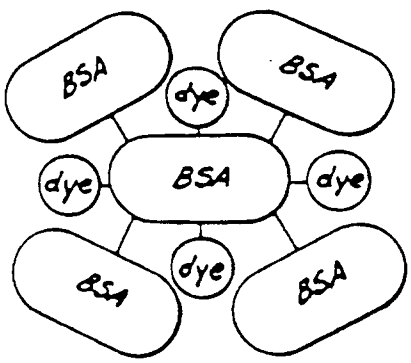


(24)

The BSA protected version (24) of the dye-BSA conjugate utilizes the chemistry described above for linking BSA conjugates and BSA-avidin conjugates. Specifically, a dye-BSA complex is treated with SMCC to yield a dye-BSA conjugate bearing maleimide groups. To this complex is added an excess of BSA bearing sulfhydryl groups; reaction with the maleimide groups on the dye-BSA conjugates results in the covalent binding of the two species to produce BSA-protected dye-BSA conjugates such as 24, which may be readily purified by size exclusion chromatography, and to which ligands such as biotin and analyte components such as 17 may be bound to produce the analyte conjugate.

BSA-blanketed conjugates such as 24 can also be used to diminish oxygen quenching of luminescence, e.g., where metalloporphyrins are used as the marker component, by sequestering such markers ("dye") from contact with ambient oxygen. For example, by shielding oxygen-quenchable luminescent markers such as platinum porphyrins (see Example 7) within the hydrophobic core of proteinaceous conjugate 24, oxygen is physically excluded from interacting with the metal in its excited state. Quenching of the luminescent signal by vibrational deactivation, such as is known to occur in lanthanide porphyrins, is analogously reduced by complexing, e.g., a ytterbium porphyrin within a protective conjugate such as 24.

EXAMPLE 7

The unique luminescent properties of the platinum (Pt) and ytterbium (Yb) porphyrin derivatives make them attractive reporting groups in immunoassay systems. Like aluminum phthalocyanine, the platinum and

ytterbium porphyrins exhibit large Stokes shifts and emit at wavelengths (650 and 975 nm, respectively) beyond those of endogenous physiological fluorophores. Unlike aluminum phthalocyanine, the emissions of Pt and Yb porphyrin are long-lived and furthermore can be selectively quenched in solution. When coupled directly or indirectly to analytes such as theophylline, the porphyrin derivatives can be used as analyte conjugates in either homogeneous or heterogeneous immunoassays. For such uses, the porphin pyrrole rings should be substituted with the aforesaid water-solubilizing groups R; representative examples are tetracarboxyltetraphenylporphyrin and tetrasulfotetraphenylporphyrin.

Homogeneous immunoassay: Upon antibody binding of either the Pt or Yb porphyrin analyte conjugate, some protection of the metallo porphyrin results. For the Pt species, the antibody provides protection from oxygen quenching of phosphorescence. In the case of the Yb derivative, the antibody serves to protect the probe from the aqueous environment and luminescence quenching by vibrational deactivation of the excited metal. As a result, in contrast to the free (and quenched) analyte conjugates present in solution, the antibody-bound analyte conjugates are luminescent. The amount of luminescence is measured and related to the amount of analyte present in the test sample.

Heterogeneous immunoassay: The analyte conjugates of the Pt and Yb porphyrins are constructed such that they are not susceptible to luminescence quenching. Luminescent derivatives require that the metal centers be surrounded by moieties that sterically prevent interactions with oxygen in the case of Pt and with water for Yb. The exemplary protected versions of the analyte conjugates 24 are luminescent in aqueous solution in the presence of oxygen. The assay quantitation then requires the separation of antibody-bound and free analyte conjugate.

Time resolution: The Pt and Yb porphyrin reagents possess luminescent lifetimes on the order of microseconds. The long-lived emission makes these probes ideal candidates for time-resolved systems. Time-gated detection of these reagents at relatively long times eliminates background fluorescence (in the nanosecond timeframe) from the matrix and the scattering events associated with excitation. The measurement of the luminescent signal against essentially no background significantly increases assay sensitivity.

EXAMPLE 8

In a representative competitive immunoassay, in which monomeric metallophthalacyanine reagents may be employed, an auxiliary dipstick is provided for conveniently sampling a predetermined volume of test fluid containing a representative aliquot of unreacted analyte conjugate and for presenting the sampled fluid portion for detection of the analyte conjugate concentration. The use of pigmented analyte conjugates containing soluble monomeric metallophthalocyanine derivatives in such an assay permits direct visual or simple instrumental readout of marker concentration in the sampled fluid portion, without signal amplification or reliance upon sophisticated electronic hardware.

Referring to FIGURE 2, a modified dipstick 10 is provided for use in a competitive immunoassay. As described in detail below, dipstick 10 is used to sample an aliquot of test fluid containing free analyte, following the addition of detectable analyte conjugate and competitive reaction with insolubilized analyte-specific binding partner, and to present the sampled fluid portion for examination and detection of the presence and concentration of unreacted analyte conjugate. Dipstick 10 is made of an insoluble, nonabsorbent material 12 such as plastic and distinctively bears a pad 14 that is capable of absorbing an aqueous test fluid. Representative absorbent or bibulous materials suitable for use in pad 14 include filter papers, nitrocellulose membranes, and silica gels. The absorbent material or materials that make up pad 14 should be white and preferably not translucent when wetted with the test fluid. Pad 14 is configured to imbibe a predetermined volume of the test fluid, including any entrained analyte conjugate, corresponding to a measurable fraction of the test fluid sample. In a representative embodiment, dipstick 10 consists of a 10cm x 1/2cm x 1mm polystyrene splint 12 bearing a 1cm x 1/2cm x 2-4mm-thick pad 14 of filter paper affixed, e.g., by an insoluble adhesive, near one end. The other end of the dipstick 10 serves as a manipulable handle. The color of the support 12 is typically not important if the pad 14 is to be instrumentally examined, such as by a reflectometer or fluorometer, but should be white or another contrasting color if the wetted pad 14 is to be visually compared to a color chart.

In use, dipstick 10 is employed in competitive immunoassays to sample a portion of test fluid containing unreacted analyte conjugate and to present that sampled fluid aliquot for visual or instrumental detection of analyte conjugate concentration. For example, the concentration of theophylline in a patient's bloodstream is measured pursuant to this disclosure as follows. First, a measured volume, on the order of 50 to 500 microliters, of the patient's blood is drawn. To this fluid sample is added and mixed a measured amount of detectable analyte conjugate made up of a theophylline molecule tethered to a colored or fluorescent dye such as the disclosed monomeric metallophthalocyanines. The mixed solution is contacted

with an insoluble substratum or carrier, such as beads on the order of 0.1 to 5 microns in diameter, bearing a measured amount of insolubilized anti-theophylline antibody or other theophylline-specific binding partner, such as Fab fragments. Such beads are preferably composed of a relatively dense material, so that the beads will settle to the bottom of the fluid sample, and may be made of insoluble plastic, glass, polysaccharide, or latex. The theophylline-specific binding partner is typically coupled to the beads or other substratum using known covalent bonding techniques. In practice, the above-indicated amounts of the theophylline conjugate and the insolubilized binding partner are empirically selected so that substantially all the conjugate binds to the beads when no free theophylline is present in the fluid sample, and so, when free theophylline is present, some conjugate remains unbound in proportion to the free theophylline concentration in the sample.

The beads are incubated and typically mixed in the fluid sample to permit competitive binding reactions to occur between the theophylline conjugate and any free theophylline for the specific binding partners on the beads. The beads are thereafter permitted to settle to the bottom of the fluid sample. Dipstick 10 is then manipulated to contact pad 14 with the supernatant above the beads for a period of time, on the order of seconds, sufficient for pad 14 to absorb the predetermined aliquot of the fluid sample containing a representative fraction of any unreacted theophylline conjugate. Dipstick 10 is removed from the fluid sample (which is then discarded along with the beads) and may be inserted into a reflectometer, fluorometer, or like instrument to detect and quantify any marker activity on the pad 14.

Referring to FIGURE 3, in another embodiment the pad 14' on dipstick 10' is a laminate composed of a porous outer membrane 16 that provides nonspecific or specific analyte-conjugate binding sites and an inner wick 18 of bibulous material, with porous membrane 16 enveloping bibulous wick 18 so that fluid communication with wick 18 must be established through membrane 16. For example, membrane 16 can be made of a material, such as nitrocellulose, that is capable of adsorbing functional groups that are generally found on most proteins. Thus, any entrained analyte conjugate (along with other proteinaceous materials in the fluid sample) will nonspecifically bind to and become concentrated on the nitrocellulose envelope 15 as the wick 18 draws a predetermined aliquot of test fluid into pad 14'.

In a preferred embodiment, the outer membrane 16 shown in FIGURE 3 is capable of specifically binding the analyte-conjugate component of the test fluid. For example, outer membrane 16 can be avidinized, in which case biotinylated analyte conjugate will specifically bind to and become concentrated on the envelope 16 as the wick 18 draws a predetermined aliquot of test fluid into pad 14'. Suitable materials for such membranes 16 include paper, nitrocellulose, nylon, polyvinilidene fluoride, and like materials that are derivatized or derivatizable to permit covalent coupling to an analyte-conjugate-specific binding partner, after which this membrane 16 should exhibit low nonspecific binding to proteins in solution. The resulting concentration of the entrained analyte conjugate on the outer surface of pad 14', by virtue of the specific binding sites on membrane 16, facilitates visual or instrumental detection and enhances the sensitivity of the disclosed assay.

In a related embodiment, the pad 14 on dipstick 10 can be provided with the analyte-conjugate-specific binding partner.

As mentioned above, analyte conjugate is a conjugated molecule having an analyte component bonded to or otherwise complexed with a detectable marker component. The analyte component can be a derivative or analog of the free analyte. The analyte conjugate by virtue of its analyte component should have substantially the same avidity as the free analyte for the insolubilized anti-analyte binding partner on the beads. In a representative embodiment of dipstick 10', membrane 16 on pad 14' is provided with the same analyte-specific binding partner as the beads or other insoluble substratum upon which the competitive binding reaction occurs. However, unlike the beads, on which the binding-partner concentration is carefully adjusted, the membrane 16 should bear an excess in the order of one to several logs of analyte-specific binding partner, compared to the projected concentrations of free analyte and unreacted analyte conjugate following the preliminary incubation step.

Alternatively, membrane 16 or pad 14 may be provided with a binding partner that is capable of specifically binding the analyte conjugate but not the free analyte. For such purposes the analyte conjugate is an analyte component bonded or otherwise complexed with a ligand/marker, and the membrane 16 or pad 14 bears a ligand/marker binding partner that does not cross-react with the analyte. As used herein, the term 'ligand/marker" refers to a molecule or molecules, or conjugated molecule, that perform(s) at least two functions when part of the analyte conjugate: Ligand/marker acts as a ligand to provide specific binding of entrained analyte conjugate to the ligand/marker binding partner on membrane 16 or pad 14. Ligand/marker also acts as a marker that can be detected by conventional techniques such as measurement of absorption, reflection, or emitted light by either the ligand/marker or by, as where the ligand/marker includes an enzyme, a reaction product of the ligand/marker. In certain embodiments, the ligand/marker may also

perform a third function by incorporating a ligand or carrier component that shelters a quenchable luminescent marker component from oxygen or vibrational deactivation.

The aforesaid functions can be performed by suitably selected ligand/markers that consist of either a single molecule, a conjugate of a ligand component bonded to a marker component, or a conjugate of a carrier component bonded to a ligand component and a marker component. As another alternative, a ligand component and a marker component can be conjugated to separate sites on an analyte component to make the analyte conjugate. As an example, the fluorescent phycobiliprotein molecule can act as a convenient ligand/marker that advantageously combines the aforesaid ligand and marker functions in a single molecule. As another example, ligand/marker can be made by conjugating a horseradish peroxidase (HPO) marker component to a ligand component such as biotin.

The marker component can be any molecule whose presence can be specifically detected on pad 14 or membrane 16, so as to thereby indicate the presence and preferably concentration of any analyte conjugate in the fluid absorbed by the pad 14 or 14'. Suitable marker components include: enzymes such as horseradish peroxidase, beta-galactosidase, glucose oxidase, and alkaline phosphatase; chromophores or organic dyes, particularly the monomeric phthalocyanines disclosed herein; fluorophores such as monomeric aluminum phthalocyanine, fluorescein, phycobiliprotein, and rhodamine; luminescent metalloporphyrins such as platinum and ytterbium porphyrin derivatives, particularly tetraphenylporphyrins and tetracarboxytetraphenylporphyrins; coenzymes such as FAD; chemiluminescent materials such as luciferins; enzyme inhibitors such as phosphonates; and radionuclides. If the marker component is an enzyme useful for colorimetric assay, the reaction product should be insoluble so that the marker activity will be sequestered on the pad 14 or membrane 16; peroxidase substrates fulfill this requirement. Such enzyme substrates and cofactors are preferably insolubilized on pad 14 or membrane 16; alternatively, after sampling test fluid containing enzyme-linked analyte conjugate, pad 14' can be dipped into a separate solution containing such color-developing reagents.

Suitable ligand components include: biotin, bovine serum albumin (BSA), synthetic peptides, phycobiliproteins, and $\beta$-galactosidase, all of which have readily available ligand/marker binding partners.

Suitable macromolecular carrier components include bovine serum albumin (BSA) and other proteins of greater than, e.g., 10,000 molecular weight. Smaller synthetic peptides and other spacer groups, including known hetero- and homobifunctional linking compounds, can also be employed as carrier components in order to focus the analyte component and the ligand/marker component(s) in specific orientation on the analyte conjugate.

In embodiments in which the free analyte is an immunogenic molecule, the analyte component of the analyte conjugate can have the same molecular structure as the analyte to be assayed or can be a metabolic derivative or a synthesized or raised analog thereof. For example, analyte component can be an anti-idiotype antibody to the analyte-specific binding site on the beads or other substratum, provided the analyte and analyte conjugate have similar binding avidity for those binding sites. In embodiments wherein the free analyte is an antibody, the analyte component can be the same or a different antibody, with the binding site on the beads having an epitope that is competitively bound by both the analyte and the analyte conjugate.

For optimal stoichiometry, as many ligand components as possible should be substituted onto the analyte conjugate while maintaining the respective activities of the analyte and any distinct marker components; in addition, the analyte conjugates should each bear a substantially uniform number of marker components. To make the analyte conjugate, ligand/marker can be conjugated to analyte component using strong ionic or covalent bonding, by diazotization, by water soluble carbodiimides, by utilizing available carboxyl groups and amino groups, and via bifunctional compounds and other chemical linkages known in the art.

The ligand/marker binding partner that is irreversibly immobilized on membrane 16 or pad 14 is typically a monoclonal or polyclonal antibody or antibody fragment having specific affinity for the ligand/marker component (but not the analyte component) of the analyte conjugate. For example, if BSA serves as the ligand component in ligand/marker, anti-BSA can serve as the ligand/marker binding partner. In a preferred embodiment, biotin serves as the ligand component in ligand/marker, and insolubilized avidin is the ligand/marker binding partner.

A test kit for practicing the invention includes the disclosed dipstick 10 or 10' in combination with a measured amount of the disclosed analyte conjugate reagent and beads or another insoluble substratum bearing a related amount of insolubilized analyte-specific binding partner. A reaction vessel capable of receiving and containing a measured volume of test fluid, in fluid communication with the analyte conjugate reagent and the analyte-specific insoluble carrier, is also typically provided, as are written instructions for employing the aforesaid components in the improved competitive immunoassay disclosed herein. Where a

colored marker component, such as the disclosed monomeric phthalocyanines, is part of the analyte conjugate provided in the kit, a chart relating color saturation of the pad 14 or 14' to marker concentration may also be provided. An instrument for reading reflectance, absorbance, or luminescence of marker component on the dipstick's pad 14 or 14' may also be included. The dipstick 10 or 10' will be configured to permit the absorbent pad 14 or 14' to be inserted into both the test-fluid-filled chamber of the reaction vessel and the light path of the instrumental reader. In place of beads, the insoluble substratum that bears the analyte-specific binding sites can take the form of, e.g., a screen or meshwork and/or can be integrated into, for example, the floor and/or sidewalls of the reaction vessel.

In a preferred embodiment, monomeric metallophthalocyanine derivatives, preferably soluble copper, silicon, and aluminum derivatives, are chemically linked to analyte components in order to provide pigmented (and, in the case of aluminum or silicon phthalocyanine, luminescent) analyte conjugates having sufficient absorptivity to permit meaningful direct measurement of very dilute analyte conjugate concentrations without further signal amplification. In addition, the monomeric copper phthalocyanine derivatives advantageously absorb at wavelengths that are directly readable by available hand-held reflectometers such as those sold under the trademark GLUCOSCAN by Lifescan, Inc., Mountain View, CA 94043.

EXAMPLE 9

Referring now to FIGURES 4 through 8, a competitive binding dipstick 44', suitable for use with the disclosed monomeric metallophthalocyanines, is provided with one or more reaction chambers 20'. As described below, reaction chamber 20' is configured to receive and direct a predetermined volume (e.g., 10 to 100 μl) of test fluid (e.g., urine or plasma) containing the targeted analyte (e.g., a drug metabolite or allergen) sequentially past and preferably through the first and second reaction surfaces 22, 24.

In the representative embodiment shown in FIGURES 5 through 7, a plurality of second reaction surfaces 24 are disposed in spaced array along a test surface 46 of an insoluble support 48. Here, each second reaction surface 24 is preferably composed of a porous binding substrate, and a hydrophilic membrane 70 of substantially the same dimensions as 24 is preferably sandwiched between the porous reaction surface 24 and the test surface 46. Each second reaction surface 24 and underlying membrane 70 are enveloped by an insoluble, impermeable reaction chamber housing 72. Housing 72 has sidewalls 74 that are positioned on and detachably mounted to the test surface 46. The sidewalls 74 should also contiguously border the lateral margins 76 of at least the second reaction surface 24 and thereby house the underlying membrane 70 in a separate compartment, so that fluid communication with membrane 70 can only be established through a porous second reaction surface 24.

A first reaction surface 22 is disposed in the distal (meaning away from surface 46) end of housing 72, and separated from second reaction surface 24 by a cavity 78 of predetermined volume. A reagent membrane 80 is positioned in the housing 72 distal to the first reaction surface 22. Distal to membrane 80, the housing 72 has an aperture 82 that opens to the environment. First reaction surface 22 and reagent membrane 80 are, like second reaction surface 24, porous to the passage of test fluid, including dissolved or entrained analyte. Sidewalls 74 similarly envelope the lateral margins 76 of first reaction surface 22 and reagent membrane 80. Thus, the distal end of housing 72 is configured so that fluid communication with cavity 78 can only be established by the passage of test fluid through aperture 82 and then sequentially through reagent membrane 80 and first reaction surface 22.

For the purpose of this discussion, analyte is considered unless otherwise stated to be an immunogenic molecule such as an antigen or hapten, and analyte binding partner is considered to be an antibody having analyte-specific binding sites. However, analyte can be any molecule for which an analyte binding partner exists or can be raised or synthesized. Thus, analyte may be an antibody and analyte binding partner can be an antigen or hapten, in which case both the analyte and the analyte component of the analyte conjugate would have binding sites that are specifically reactive with the immunogenic analyte binding partner immobilized on the first reaction surface 22.

Test fluid sample can be any aqueous source of analyte; for example, physiological fluids such as anti-coagulated blood, plasma, urine, tissue extract, or saliva, in which case the analyte can be any endogenous or exogenous molecule, such as a hormone or a therapeutic or abused drug and their metabolites. Test fluid must be aqueous or largely so, containing only small amounts of organic solvents so that analyte binding partner and analyte conjugate will not spontaneously dissociate in the test fluid sample. In this regard, the analyte must also be available in aqueous solution for binding with analyte binding partner. Thus, to detect serum protein-bound hormones such as cortisol, detergent can be added to the test fluid sample in order to release the hormone analyte from the protein carrier and thereby make it fully available to an analyte binding partner having cortisol-specific binding sites. Such detergents and other agents, where

applicable, can be placed on the inner sidewalls 74, e.g., by evaporation or impregnation, prior to packaging the reaction chamber 20', in order to maintain the simplicity of the disclosed assay protocol.

Reaction chamber 20' is configured to receive a test fluid sample and retain the same (including any displaced analyte conjugate) throughout the incubation period. For stoichiometrical determinations of analyte concentration, reaction chambers of uniform volume should be provided. The housing 72 that contains the test fluid sample within the reaction chamber 20' can be made of any insoluble material, e.g., plastic, nylon, polypropylene, polyvinylchloride, or polyvinylcarbonate, that does not react with either analyte or displaced analyte conjugate. The reaction chambers 20' can also take the form of test tubes or microtiter wells, as described below.

First reaction surface 22 and second reaction surface 24 are each composed of a binding substrate, which can be a molecular film, having the capacity to bind reactants via covalent or strong ionic bonds such that analyte binding partner and ligand/marker binding partner can be irreversibly immobilized on the first and second reaction surfaces 22, 24, respectively. Suitable binding substrates for this purpose include derivatized celluloses such as nitrocellulose, bromacetyl cellulose, cyanogen bromide activated cellulose, and also derivatized nylon, derivatized plastics, and other activated polymers.

Analyte binding partner is preferably a monoclonal antibody having at least one binding site that is specifically reactive to analyte and to analyte component. Analyte binding partner can be a divalent antibody or an antibody polymer (or polymerized Fab fragments) having a plurality of analyte-specific binding sites. However, it is contemplated that steric hindrance between the available analyte-specific binding sites should be minimized for stoichiometrical quantification purposes, in which case a monovalent antibody or Fab fragment is the preferred analyte binding partner. Analyte binding partner can be covalently bonded to first reaction surface 22 by conventional techniques; for example, by conventional reactions involving carbonyl groups, carboxyl groups, the $\epsilon$-amino acid groups of lysine, or the SH groups of cysteine, by strong ionic interactions, and by specific interaction with protein A. A multiplicity of analyte binding partners are bound to the first reaction surface so that analyte-specific binding sites are thereafter available for binding with analyte or analyte component in log excess over the expected concentration of analyte in the test fluid sample. To this end, through the use of heterobifunctional chemical compounds and through other chemical reactions known in the art, analyte binding partner can be immobilized on first reaction surface 22 in specific orientation in order to make analyte-specific binding site available to analyte component of analyte conjugate and to analyte in test fluid sample. Moreover, the accessibility with regard to steric hindrance of available analyte-specific binding sites can be enhanced by selectively distributing analyte binding partner on the first reaction surface 22. Optimal density of particular analyte binding partners can be achieved by selective chemical activation of the first reaction surface 22 or by using immunoaffinity membranes 22 that are commercially available, e.g., BIODYNE immunoaffinity membrane (PALL, Biodyne Division, Glen Cove, NY).

Analyte conjugate is a conjugated molecule: analyte component bonded to ligand/marker. Ligand/marker is a molecule or molecules or conjugated molecule that perform(s) two functions: Ligand/marker acts as a ligand to provide specific binding of displaced analyte conjugate to ligand/marker binding partner at the second reaction surface 24. Ligand/marker also acts as a marker that can be detected by conventional techniques such as enzymatic color development. For certain applications ligand/marker may also perform a third function by acting as a macromolecular carrier in order to ensure the retention of displaced analyte conjugate within the reaction chamber 20'.

The aforesaid functions can be performed by ligand/markers that consist of either a single molecule, a conjugate of a ligand component bonded to a marker component, a conjugate of a carrier component bonded to both a ligand component and a marker component, or a ligand component and marker component can be individually conjugated at separate sites to the analyte component; all as described in Example 8.

Ligand/marker binding partner can be a monoclonal or polyclonal antibody having specific affinity for ligand/marker component (but not for analyte component) of analyte conjugate. For example, if BSA serves as the ligand component in ligand/marker, then anti-BSA can serve as the ligand/marker binding partner. As another example, if biotin serves as the ligand component in ligand/marker, then ligand/marker binding partner can be insolubilized avidin. Other representative but nonlimiting examples of ligand/marker binding partners include second partners include second partners of activated heterobifunctional substituted ligand/markers. Ligand/marker binding partner is irreversibly immobilized on second reaction surface 24 so that ligand/marker-specific binding sites are available for binding to ligand/marker in log excess of the expected concentration of analyte (and, proportionally, of displaced analyte conjugate) in test fluid sample.

Analyte binding partner and ligand/marker binding partner are bound to the first and second reaction surfaces 22, 24, respectively. For use in competitive binding reaction chambers 20', the association

constants of binding partner, with reference to the free analyte and the analyte component of analyte conjugate, may be identical. Suitable porous binding substrates for reaction surfaces 22, 24 include the activated microporous membranes sold under the trademark IMMOBILON by the Millipore Corporation.

Reagent membrane 80 contains solubilizable analyte conjugate. As discussed above, the analyte conjugate is preferably a conjugate of the analyte with a ligand/marker made up of, for example, a marker (e.g., a colored dye) and a ligand (e.g., biotin).

In use, the dipstick 44' including at least one reaction chamber 20' is submerged in a test fluid containing an unknown amount of analyte. The chamber 20' is incubated for a short period of time, on the order of minutes or less, sufficient for test fluid to contact and pass sequentially through porous films 80, 22 and 24. Bibulous membrane 70 acts as a wick to drive this one-way flow. To release entrapped gas, the sidewalls 74 can be provided with one or more auxiliary openings 84, each screened with a hydrophobic but gas-permeable mesh. Analyte conjugate is solubilized as the test fluid passes through film 80. At the first reaction surface 22, competition between analyte and analyte conjugate for analyte-specific binding sites permits passage and contact with the second reaction surface 24 of analyte analog in proportion to the analyte concentration in the test fluid. The ligand/marker component of the analyte conjugate becomes bound to the ligand/marker-specific binding sites on the second reaction surface 24, producing a detectably marked surface indicative of analyte presence in the test fluid. Readout can be immediately achieved by, for example, pulling tab 86 to rupture housing 72 and expose the second reaction surface 24 for visual or instrumental inspection.

Alternatively, as shown in FIGURE 8, an aperture or transparent window 88 can be provided in dipstick 44' on the proximal side of the second reaction surface 24, for examination and detection of sequestered marker by absorption, reflected or emitted light. In such an embodiment, the second reaction surface 24 can take the form of a transluscent and preferably transparent sheet of nitrocellulose or filter paper, avidinized on its distal surface. An opaque, and preferably white or otherwise reflective, porous membrane 90 may be disposed between the two reaction surfaces 22, 24 in order to shield any marker bound to the first surface 22 from view through window 88.

By providing predetermined quantities of the analyte conjugate and the analyte binding sites in porous films 80 and 22, respectively, and by providing chambers 20' of uniform fluid capacity, the concentration of analyte conjugate that binds to the second reaction surface 24 can be used to quantify the analyte concentration in the test fluid.

The dipstick 44' may advantageously be provided with a plurality of chambers 20' having identical second reaction surfaces 24 but different analyte binding sites and analyte conjugates. Dipstick 44' can thus be an immediate, one-step, multi-analyte readout, including suitable positive and negative controls.

Referring now to FIGURES 9 and 10, a modified microtiter well 92 having a test fluid volume capacity of 100 to 200 µl can also serve as a reaction chamber 20'. A first reaction surface 22 can be coaxially disposed on the sidewall 94 of the well 92, and a planar second reaction surface 24 can be disposed to cover all or part of the floor 96 of the well 92. In the competitive binding chamber 20', the analyte conjugate may be reversibly sequestered in solubilized form on the sidewall 94 or floor 96 of the well, apart from the reaction surfaces 22, 24. Such reaction chambers 20' can be conveniently filled with a test fluid sample using an automatic pipette. Following an incubation period, any marker activity on the second reaction surface 24 can be conveniently read using, for example, an ELISA plate reader. A ring of containment wall 21 can be provided at the base of sidewall 94 so that substrate can be added to cover the second reaction surface 24 without contacting the first reaction surface 22. This embodiment of chamber 20' is particularly well suited for repetitive laboratory testing by technical personnel using sophisticated instrumentation. For example, a plate 98 can be provided with a plurality of reaction chambers 20' for each test fluid to be assayed; some of the reaction chambers can be filled with replicate test fluid samples, and other reaction chambers can be filled with control solutions containing known concentrations of the analyte or analytes being assayed.

Referring to FIGURES 11 and 12, in a related embodiment a modified test tube 100 can also serve as a reaction chamber 20'. For example, first and second reaction surfaces 22, 24 can be disposed in nonoverlapping arrangement on the inner wall 102 of a test tube 100. In the competitive binding chamber 20', analyte conjugate may be reversibly sequestered on the inner wall 102 apart from either reaction surfaces 22, 24. During the incubation period the test fluid sample (not shown) can be periodically stirred or vortexed in order to effect contacts between the reagents and the reaction surfaces 22, 24. If the first and second reaction surfaces 22, 24 are not diametrically opposed on the test tube wall 102, then colorimetric or fluorometric marker activity on the second reaction surface 24 can be conveniently read by inserting tube 100 into a standard spectrophotometer or fluorometer.

While the preferred embodiments of the invention have been illustrated and described, it is to be understood that, within the scope of the appended claims, various changes can be made therein. Hence, the invention can be practiced in ways other than those specifically described herein.

## Claims

1. A reagent composition comprising a physiological analyte and a phthalocyanine derivative, the phthalocyanine derivative being conjugated in monomeric form in the reagent composition.

2. A reagent composition as claimed in Claim 1, wherein the phthalocyanine derivative is metallated with aluminium, copper, silicon, phosphorus, gallium, germanium, cadmium, magnesium, tin, or zinc.

3. A reagent composition as claimed in any of Claims 1 to 2, wherein the phthalocyanine derivative comprises one or more substituents which is sulphonic acid, sulphonate, carboxylic acid, carboxylate, phosphoric acid, phosphate, phosphonate, hydroxy, phenoxy, amino, ammonium, or pyridinium.

4. A reagent composition as claimed in any of Claims 1 to 3, wherein the phthalocyanine derivative is conjugated in monomeric form via an enzyme-cleavable linkage with the physiological analyte.

5. A reagent composition as claimed in any of Claims 1 to 4, which encompasses a complex of a monomeric phthalocyanine derivative that is conjugated to, and substantially enveloped by, a hydrophilic component, like bovine serum albumin, of the reagent composition.

6. A reagent composition as claimed in any of Claims 1 to 5, wherein the physiological analyte is a drug, drug metabolite, hormone, peptide, nucleotide, neurotransmitter, cholesterol, growth factor, oligonucleotide, antibody, antigen-binding fragment, serum protein, enzyme, polynucleotide, intracellular organelle, cell surface antigen, avidin or biotin.

7. A reagent composition for use in an enzymatic assay comprising a phthalocyanine derivative conjugated in monomeric form via an enzyme-cleavable linkage with a moiety comprising an atom having an atomic weight greater than 80, a paramagnetic atom, or a paramagnetic group.

8. A reagent composition for use in an enzymatic assay comprising a first phthalocyanine conjugated in monomeric form via an enzyme-cleavable linkage with a second phthalocyanine.

9. A reagent composition comprising a complex of an oxygen quenchable luminescent compound conjugated with a hydrophilic carrier molecule, like bovine serum albumin, the carrier molecule conjugated so that it envelopes the luminescent compound of the complex to render the luminescent compound substantially unreactive to ambient oxygen.

10. A reagent composition comprising a complex of a vibration quenchable luminescent compound conjugated with a hydrophilic carrier molecule, like bovine serum albumin, the carrier molecule conjugated so that it envelopes the luminescent compound of the complex to substantially prevent collision of ambient small molecules with the luminescent compound.

11. A kit useful for detecting the presence of an analyte in a fluid sample, comprising:

    a measured amount of an analyte conjugate, which is a phthalocyanine derivative in a monomeric form, comprising an analyte component and a detectable marker component;

    a first insoluble carrier having immobilized thereon a measured amount of an analyte-specific binding partner;

    a reaction vessel capable of receiving and containing, in fluid communication with the measured amount of analyte conjugate and the first insoluble carrier, a measured volume of a fluid sample suspected of containing an analyte; and,

    a second insoluble carrier having a manipulable first end and a second end bearing a pad of bibulous material capable of absorbing a predetermined volume of the fluid sample, the second end being configured to be received by the reaction vessel in order to contact the pad with the fluid sample.

12. A kit as claimed in Claim 11 wherein the pad of bibulous material is capable of binding the analyte conjugate.

13. A kit as claimed in any of Claims 11 or 12 wherein the pad of bibulous material is enveloped by a porous membrane capable of binding the analyte conjugate.

14. A device for detecting an analyte in a test fluid, comprising a reaction chamber having reagent, reaction, and detection surfaces wherein at least the detection surface does not overlap either the reagent surface or the reaction surface, the reaction chamber being adapted to receive and retain a predetermined volume of the test fluid in fluid communication with the reagent, reaction, and detection surfaces, the reagent surface having an analyte conjugate, which comprises a phthalocyanine derivative in monomeric form, reversibly bound thereto, the analyte conjugate comprising a ligand/marker conjugated to an analyte component, the reaction surface having an analyte binding partner immobilized thereon, and the detection surface having a binding partner for the ligand/marker immobilized thereon.

15. A device as claimed in Claim 14 wherein the reaction chamber is adapted to receive and direct the test fluid sequentially past the reagent, reaction and detection surfaces.

## Patentansprüche

1. Reagenzzusammensetzung, umfassend einen physiologischen Analyt und ein Phthalocyaninderivat, wobei das Phthalocyanin-Derivat in monomerer Form in der Reagenzzusammensetzung konjugiert vorliegt.

2. Reagenzzusammensetzung nach Anspruch 1, wobei das Phthalocyaninderivat mit Aluminium, Kupfer, Silicium, Phosphor, Gallium, Germanium, Cadmium, Magnesium, Zinn oder Zink mtalliert ist.

3. Reagenzzusammensetzung nach einem der Ansprüche 1 bis 2, wobei das Phthalocyaninderivat ein oder mehrere Substituenten umfaßt, nämlich Sulfonsäure-, Sulfonat-, Carbonsäure-, Carboxylat-, Phosphorsäure-, Phosphat-, Phosphonat-, Hydroxy-, Phenoxy-, Amino-, Ammonium- oder Pyridiniumgruppen.

4. Reagenzzusammensetzung nach einem der Ansprüche 1 bis 3, wobei das Phthalocyaninderivat in monomerer Form über eine enzymspaltbare Bindung mit dem physiologischen Analyt konjugiert vorliegt.

5. Reagenzzusammensetzung nach einem der Ansprüche 1 bis 4, umfassend einen Komplex eines monomeren Phthtalocyaninderivats, das konjugiert ist mit und im wesentlichen umhüllt ist von einer hydrophilen Komponente der Reagenzzusammensetzung, wie Rinderserumalbumin.

6. Reagenzzusammensetzung nach einem der Ansprüche 1 bis 5, wobei der physiologische Analyt ein Arzneistoff, Arzneistoffmetabolit, Hormon, Peptid, Nucleotid, Neurotransmitter, Cholesterin, Wachstumsfaktor, Oligonucleotid, Antikörper, Antigen-bindendes Fragment, Serumprotein, Enzym, Polynucleotid, intrazelluläre Organelle, Zelloberflächenantigen, Avidin oder Biotin.

7. Reagenzzusammensetzung zur Verwendung in einem Enzymtest, umfassend ein Phthalocyaninderivat, das in monomerer Form über eine enzymspaltbare Bindung mit einer Einheit konjugiert ist, welche ein Atom mit einem Atomgewicht über 80, ein paramagnetisches Atom oder eine paramagnetische Gruppe umfaßt.

8. Reagenzzusammensetzung zur Verwendung in einem Enzymtest, umfassend ein erstes Phthtalocyanin, das in monomerer Form über eine enzymspaltbare Bindung mit einem zweiten Phthalocyanin konjugiert ist.

9. Reagenzzusammensetzung, umfassend einen Komplex einer Sauerstoff-löschbaren lumineszierenden Verbindung, die mit einem hydrophilen Trägermolekül konjugiert ist, wie Rinderserumalbumin, wobei das Trägermolekül so konjugiert ist, daß es die lumineszierende Verbindung des Komplexes so

umhüllt, daß die luimineszierende Verbindung im wesentlichen gegenüber Sauerstoff aus der Umgebung nicht reaktiv ist.

**10.** Reagenzzusammensetzung, umfassend einen Komplex einer Schwingungs-löschbaren lumineszierenden Verbindung, die mit einem hydrophilen Trägermolekül konjugiert ist, wie Rinderserumalbumin, wobei das Trägermolekül so konjugiert ist, daß es die lumineszierende Verbindung des Komplexes so umhült, daß es die Kollision von kleinen Molekülen aus der Umgebung mit der lumineszierenden Verbindung im wesentlichen verhindert.

**11.** Kit, der für den Nachweis der Gegenwart eines Analyt in einer Flüssigkeitsprobe wertvoll ist, umfassend: eine abgemessene Menge eines Analytkonjugats, das ein Phthalocyaninderivat in einer monomeren Form ist, umfassend eine Analytkomponente und eine nachweisbare Markerkomponente;
einen ersten unlöslichen Träger mit einer darauf immobilisierten abgemessenen Menge eines Analyt-spezifischen Bindungspartners;
ein Reaktionsgefäß, das, in flüssiger Verbindung mit der abgemessenen Menge eines Analytkonjugats und dem ersten unlöslichen Träger, ein abgemessenes Volumen einer Flüssigkeitsprobe, die vermutlich einen Analyt enthält, aufnehmen und enthalten kann; und
einen zweiten unlöslichen Träger mit einem manipulierbaren ersten Ende und einem zweiten Ende, das ein Kissen aus saugfähigem Material aufweist, das ein vorbestimmtes Volumen der Flüssigkeitsprobe absorbieren kann, wobei das Zweite Ende so gestaltet ist, daß es von dem Reaktionsgefäß aufgenommen werden kann, so daß das Kissen mit der Flüssigkeitsprobe in Kontakt kommt.

**12.** Kit nach Anspruch 11, wobei das Kissen aus saugfähigem Material zur Bindung des Analytkonjugats fähig ist.

**13.** Kit nach einem der Ansprüche 11 oder 12, wobei das Kissen aus saugfähigem Material mit einer porösen Membran umhüllt ist, die zur Bindung des Analytkonjugats fähig ist.

**14.** Vorrichtung zum Nachweis eines Analyts in einer Testflüssigkeit, umfassend eine Reaktionskammer mit Reagenz-, Reaktions- und Nachweisoberflächen, wobei mindestens die Nachweisoberfläche weder mit der Reagenzoberfläche noch mit der Reaktionsoberfläche überlappt, wobei die Reaktionskammer so angepaßt ist, daß sie ein vorbestimmtes Volumen der Testflüssigkeit in flüssiger Verbindung mit den Reagenz-, Reaktions- und Nachweisoberflächen aufnehmen und behalten kann, wobei die Reagenzoberfläche ein Analytkonjugat aufweist, welches ein Phthalocyaninderivat in monomerer Form reversibel daran gebunden hat, wobei das Analytkonjugat einen Ligand/Marker konjugiert an eine Analytkomponente aufweist, wobei die Reaktionsoberfläche einen Analytbindungspartner immobilisiert daran aufweist und die Nachweisoberfläche ein Bindungspartner für den Ligand/Marker immobilisiert daran aufweist.

**15.** Vorrichtung nach Anspruch 14, wobei die Reaktionskammer so angepaßt ist, daß sie die Testflüssigkeit aufnimmt und nacheinander an den Reagenz-, Reaktions- und Nachweisoberflächen vorbeileitet.

## Revendications

**1.** Composition réactive comprenant un analyte physiologique et un dérivé de phtalocyanine, le dérivé de phtalocyanine étant conjugué sous sa forme monomère dans la composition réactive.

**2.** Composition réactive selon la revendication 1, dans laquelle le dérivé de phtalocyanine est métalé par de l'aluminium, du cuivre, du silicium, du phosphore, du gallium, du germanium, du cadmium, du magnésium, de l'étain ou du zinc.

**3.** Composition réactive selon l'une des revendications 1 à 2, dans laquelle le dérivé de phtalocyanine comporte un ou plusieurs substituants de type acide sulfonique, sulfonate, acide carboxylique, carboxylate, acide phosphorique, phosphate, phosphonate, hydroxyle, phénoxy, amino, ammonium ou pyridinium.

**4.** Composition réactive selon l'une des revendications 1 à 3, dans laquelle le dérivé de phtalocyanine est conjugué sous sa forme monomère via un lien clivable par une enzyme avec un analyte physiologique.

5. Composition réactive selon l'une des revendications 1 à 4, qui comporte un complexe d'un dérivé de phtalocyanine monomère qui est conjugué et qui est substantiellement enveloppé par un constituant hydrophile, tel que de l'albumine de sérum bovin, de la composition réactive.

6. Composition réactive selon l'une des revendications 1 à 5, dans laquelle l'analyte physiologique est un médicament, un métabolite de médicament, une hormone, un peptide, un nucléotide, un neurotransmetteur, du cholestérol, un facteur de croissance, un oligonucléotide, un anticorps, un fragment pouvant se lier à un antigène, une protéine de sérum, une enzyme, un polynucléotide, un organite intracellulaire, un antigène superficiel de cellule, de l'avidine, de la biotine.

7. Composition réactive destinée à être utilisée dans un test enzymatique, comportant un dérivé de phtalocyanine conjuguée sous sa forme monomère via un lien clivable par une enzyme avec une partie comportant un atome dont le poids atomique est supérieur à 80, un atome paramagnétique ou un groupe paramagnétique.

8. Composition réactive destinée à être utilisée dans un test enzymatique comportant une première phtalocyanine conjuguée sous sa forme monomère via un lien clivable par une enzyme avec une seconde phtalocyanine.

9. Composition réactive comportant un complexe d'un composé luminescent pouvant être étouffé par l'oxygène, avec une molécule porteuse hydrophile, telle que de l'albumine de sérum bovin, la molécule porteuse étant conjuguée de telle sorte qu'elle enveloppe le composé luminescent du complexe de manière à rendre le composé luminescent substantiellement non réactif vis-à-vis de l'oxygène ambiant.

10. Composition réactive comportant un complexe d'un composé luminescent pouvant être étouffé par vibrations, conjugué avec une molécule porteuse hydrophile, telle que de l'albumine de sérum bovin, la molécule porteuse étant conjuguée de telle sorte qu'elle enveloppe le composé luminescent du complexe de manière à empêcher substantiellement la collision de petites molécules environnantes avec le composé luminescent.

11. Kit utilisable pour la détection de la présence d'un analyte dans un échantillon liquide, comportant :
   - une quantité mesurée d'un produit de conjugaison de l'analyte, qui est un dérivé de phtalocyanine sous forme monomère, comportant un composant analyte et un composant marqueur détectable,
   - un premier élément porteur insoluble sur lequel est immobilisé une quantité mesurée d'un partenaire liant spécifique à l'analyte,
   - un récipient de réaction capable de recevoir et contenant, en communication liquide avec la quantité mesurée de produit de conjugaison d'analyte et le premier élément porteur insoluble, un volume mesuré d'échantillon liquide soupçonné de contenir un analyte et
   - un second élément porteur insoluble possédant une première extrémité manipulable et une seconde extrémité portant un tampon en matériau absorbant capable d'absorber un volume prédéterminé d'échantillon liquide, la seconde extrémité ayant une configuration lui permettant d'être mise en contact avec le récipient de réaction afin de mettre en contact le tampon avec l'échantillon liquide.

12. Kit selon la revendication 11, dans lequel le tampon de matériau absorbant est capable de lier le produit de conjugaison de l'analyte.

13. Kit selon la revendication 11 ou 12, dans lequel le tampon de matériau absorbant est enveloppé par une membrane poreuse capable de lier le produit de conjugaison de l'analyte.

14. Dispositif destiné à la détection d'un analyte dans un liquide à tester, comprenant une chambre de réaction possédant des surfaces de réactif, de réaction et de détection où au moins la surface de détection ne chevauche ni la surface de réactif ni la surface de réaction, la chambre de réaction étant adaptée pour recevoir et retenir un volume prédéterminé de liquide à tester en communication liquide avec les surfaces de réactif, de réaction et de détection, la surface de réactif portant un produit de conjugaison de l'analyte, qui comporte un dérivé de phtalocyanine sous forme monomère, qui lui est lié de manière réversible, le produit de conjugaison de l'analyte comportant un ligand/marqueur conjugué

à un composant de l'analyte, la surface de réaction portant un partenaire liant pour l'analyte immobilisé sur elle et la surface de détection ayant un partenaire liant du ligand/marqueur immobilisé sur elle.

**15.** Dispositif selon la revendication 14, dans lequel la chambre de réaction est adaptée pour recevoir et diriger le liquide à tester successivement sur les surfaces de réactif, de réaction et de détection.

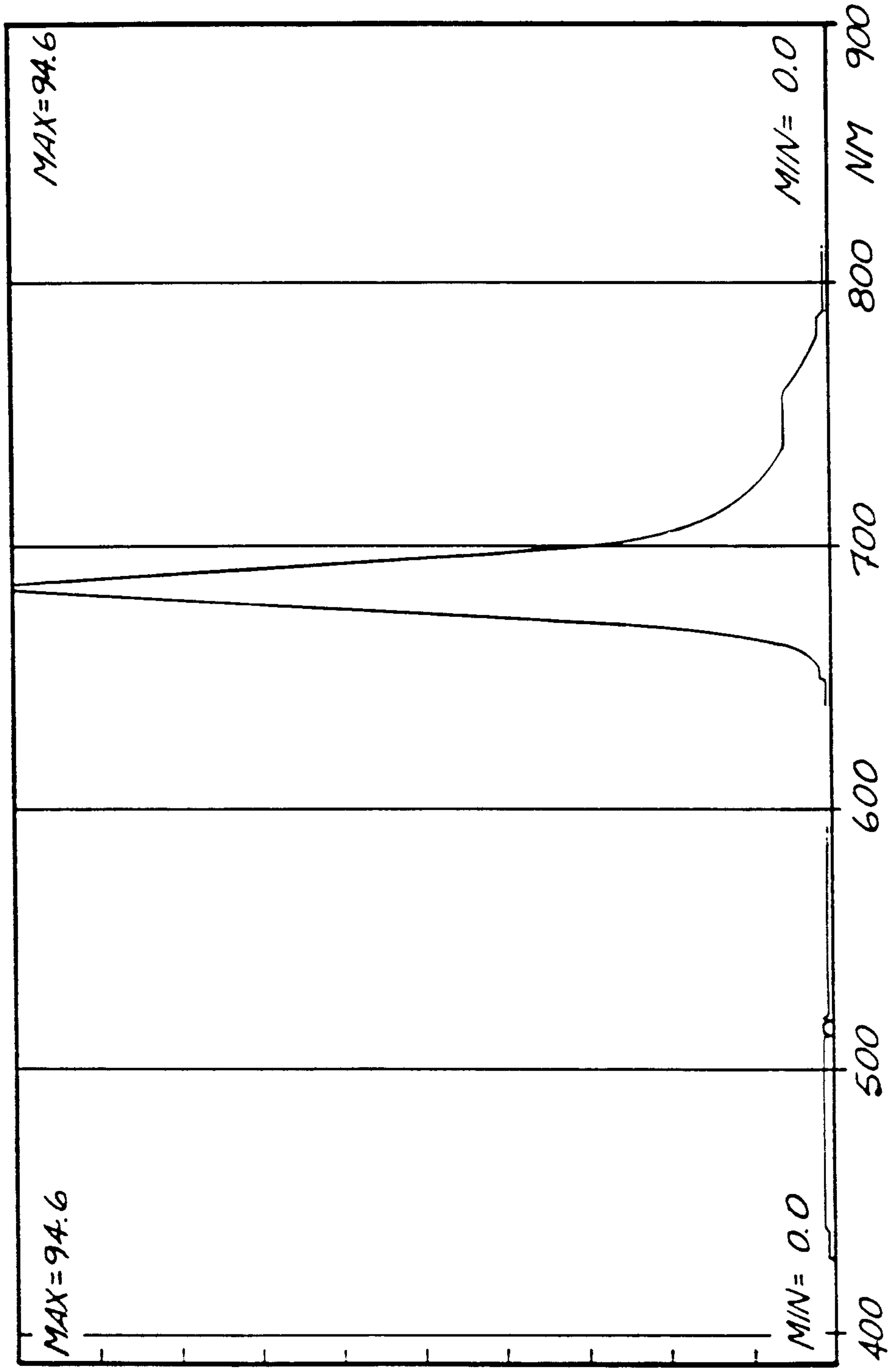


Fig.1A.

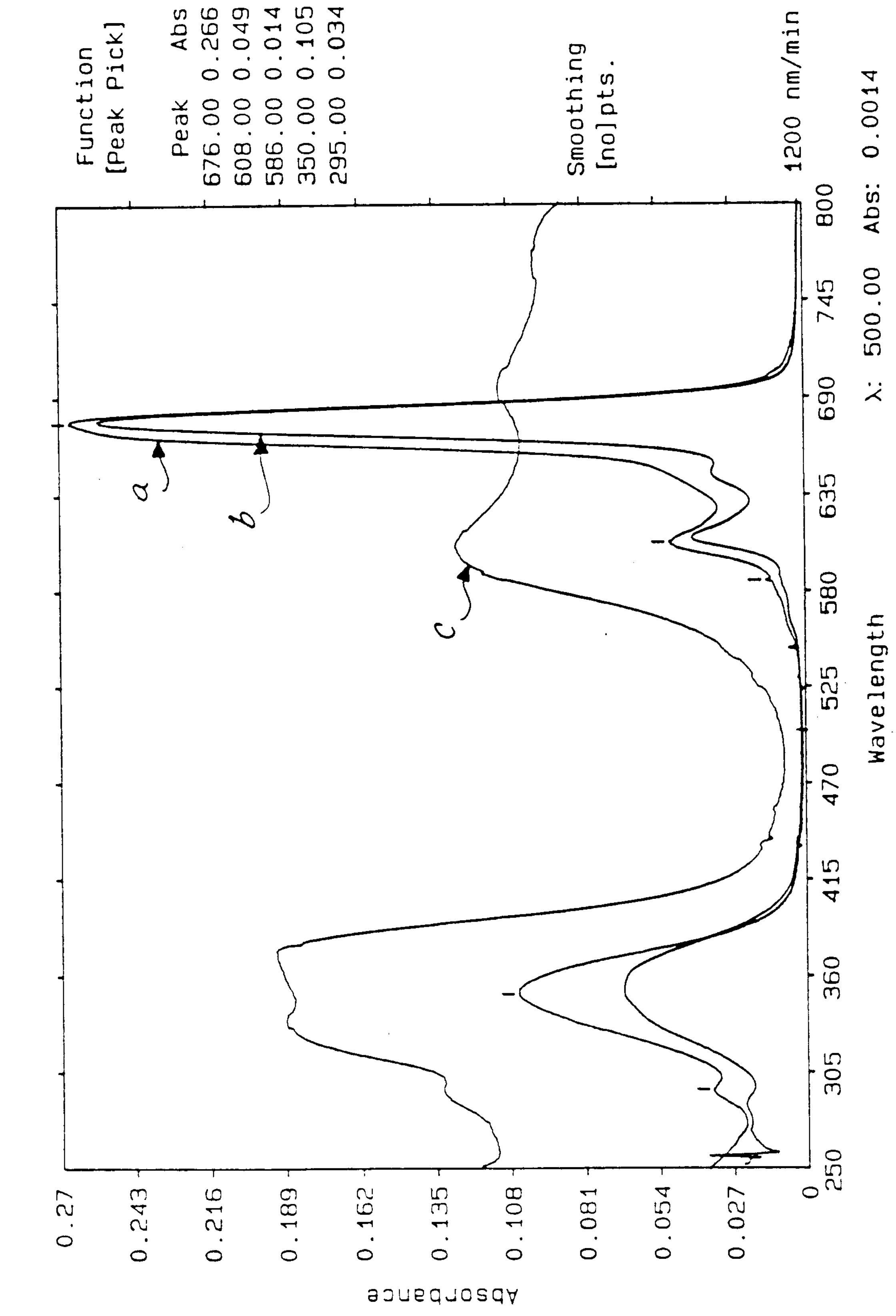
Function
[Peak Pick]
Peak Abs
676.00 0.266
608.00 0.049
586.00 0.014
350.00 0.105
295.00 0.034
Smoothing
[no]pts.
1200 nm/min
λ: 500.00 Abs: 0.0014
Absorbance
Wavelength
0.27
0.243
0.216
0.189
0.162
0.135
0.108
0.081
0.054
0.027
0
250
305
360
415
470
525
580
635
690
745
800
a
b
c

Fig.1B.

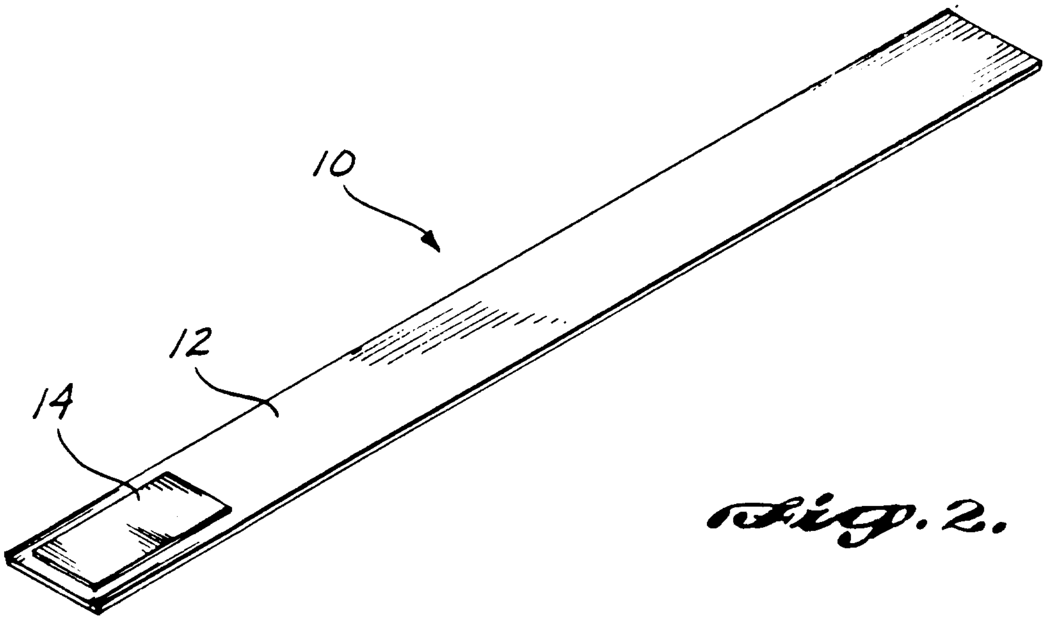


Fig. 2.

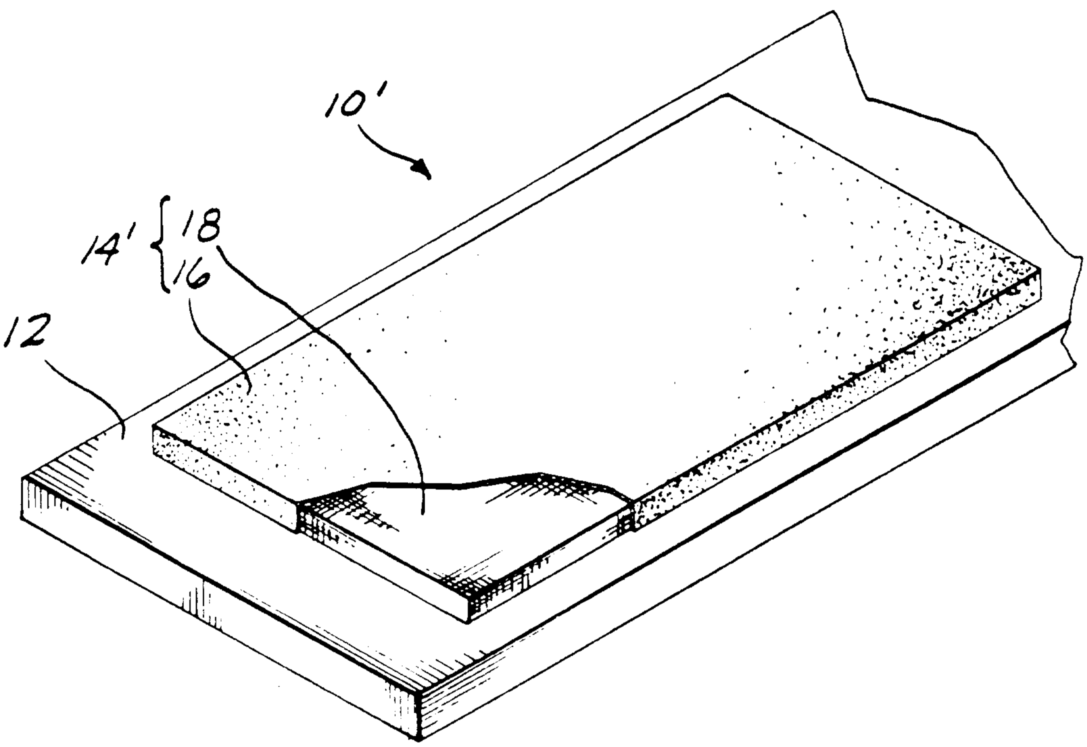


Fig. 3.

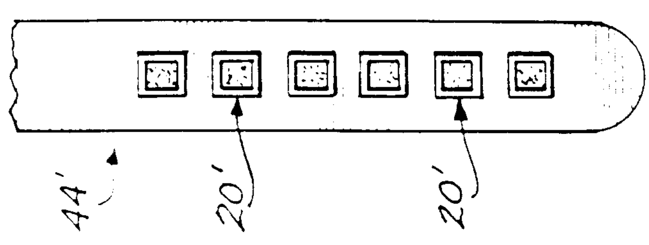

Fig. 4.

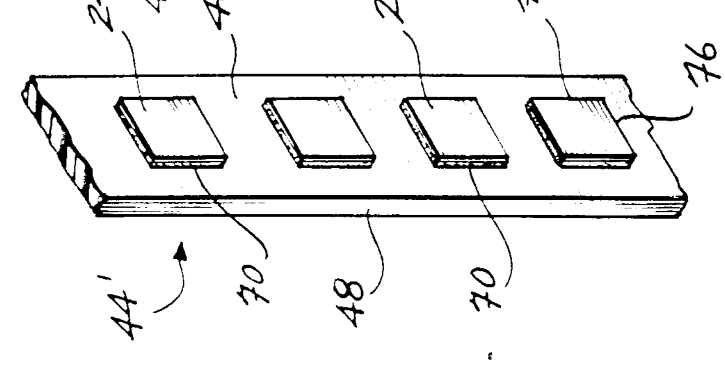

Fig. 5.

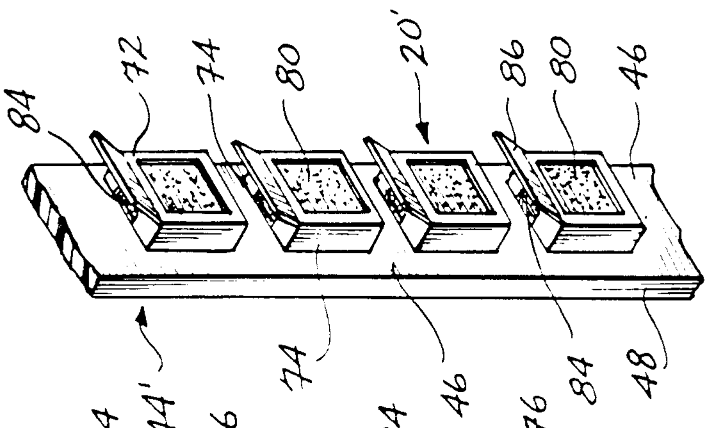

Fig. 6.

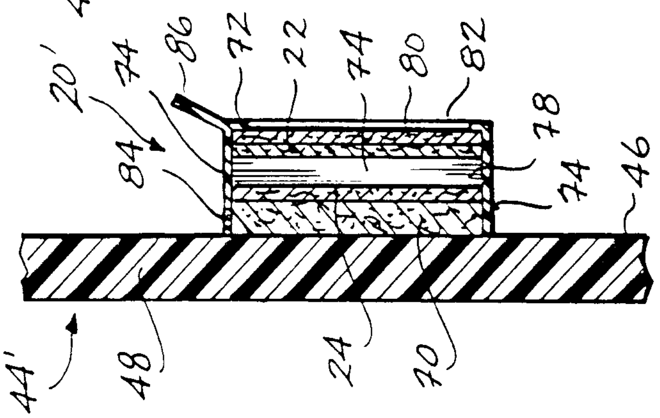

Fig. 7.

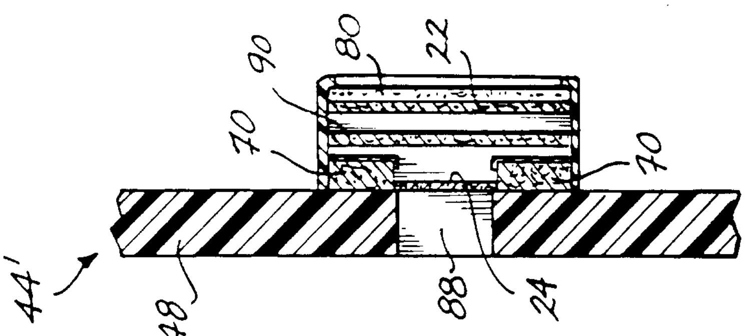

Fig. 8.

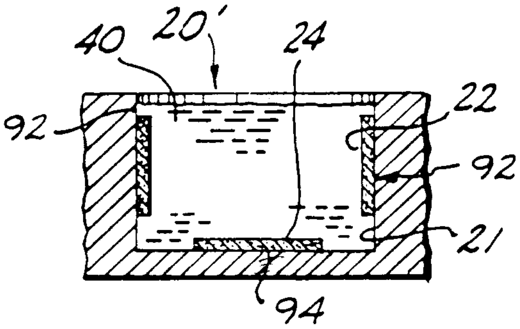


Fig. 9.

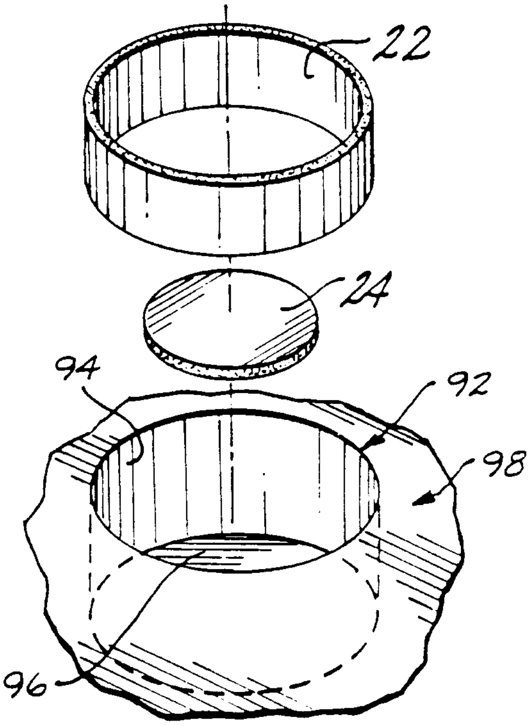


Fig. 10.

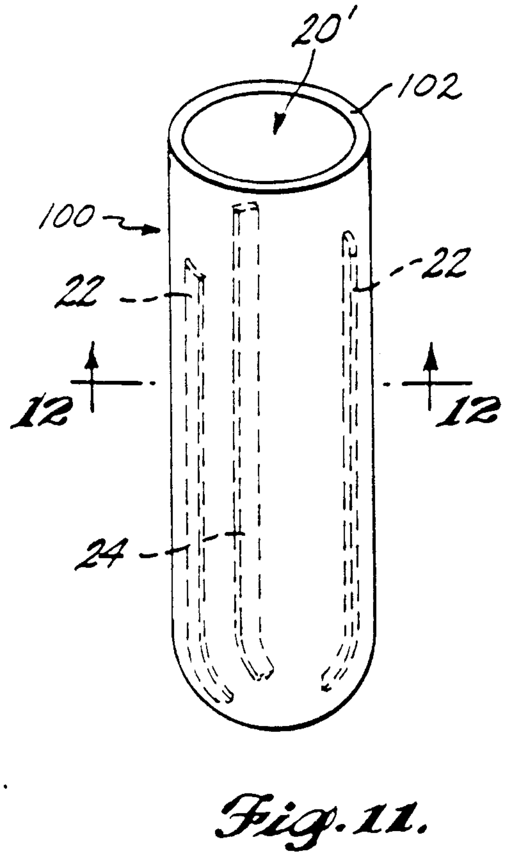


Fig. 11.

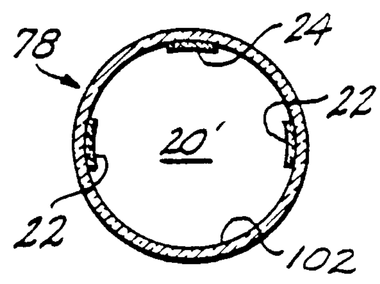


Fig. 12.